(19) Europäisches Patentamt / European Patent Office / Office européen des brevets

(11) **EP 2 266 626 B1**

(12) **EUROPEAN PATENT SPECIFICATION**

(45) Date of publication and mention of the grant of the patent:
**18.06.2014 Bulletin 2014/25**

(51) Int Cl.:
**A61K 48/00** (2006.01)   **A61K 9/06** (2006.01)
**A61K 31/7088** (2006.01)   **A61K 47/12** (2006.01)
**A61K 47/14** (2006.01)   **A61K 47/18** (2006.01)
**A61P 11/06** (2006.01)   **A61P 17/00** (2006.01)
**A61P 19/02** (2006.01)   **A61P 29/00** (2006.01)
**A61P 37/08** (2006.01)   **A61K 9/00** (2006.01)

(21) Application number: **09724284.6**

(22) Date of filing: **27.03.2009**

(86) International application number:
**PCT/JP2009/056369**

(87) International publication number:
**WO 2009/119836 (01.10.2009 Gazette 2009/40)**

(54) **COMPOSITION FOR EXTERNAL APPLICATION COMPRISING TRANSCRIPTION FACTOR DECOY AS ACTIVE INGREDIENT**

ZUSAMMENSETZUNG ZUR TOPISCHEN ANWENDUNG MIT EINEM TRANSKRIPTIONSFAKTOR-DECOY ALS WIRKSTOFF

COMPOSITION POUR APPLICATION EXTERNE COMPRENANT UN LEURRE DE FACTEUR DE TRANSCRIPTION EN TANT QU' INGRÉDIENT ACTIF

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **28.03.2008 JP 2008088801**

(43) Date of publication of application:
**29.12.2010 Bulletin 2010/52**

(73) Proprietors:
• **AnGesMG, Inc.**
  **Ibaraki-shi**
  **Osaka 567-0085 (JP)**
• **MEDRx Co., Ltd.**
  **Higashikagawa-shi**
  **Kagawa 769-2712 (JP)**

(72) Inventors:
• **SHINOHARA, Kazuya**
  **Higashikagawa-shi**
  **Kagawa 769-2712 (JP)**
• **HAMAMOTO, Hidetoshi**
  **Higashikagawa-shi**
  **Kagawa 769-2712 (JP)**
• **KOBAYASHI, Katsunori**
  **Higashikagawa-shi**
  **Kagawa 769-2712 (JP)**
• **NAKANO, Tatsuro**
  **Higashikagawa-shi**
  **Kagawa 769-2712 (JP)**
• **SAKAGUCHI, Makoto**
  **Ibaraki-shi**
  **Osaka 567-0085 (JP)**

(74) Representative: **Gille Hrabal**
  **Brucknerstrasse 20**
  **40593 Düsseldorf (DE)**

(56) References cited:
EP-A1- 1 362 600   WO-A1-96/04902
JP-A- 8 175 972   JP-A- 9 208 463
JP-A- 2006 089 475   JP-A- 2008 184 402
US-A- 5 795 916

• MANN M J ET AL: "Therapeutic applications of transcription factor decoy oligonucleotides", JOURNAL OF CLINICAL INVESTIGATION, AMERICAN SOCIETY FOR CLINICAL INVESTIGATION, US, vol. 106, no. 9, 1 November 2000 (2000-11-01), pages 1071-1075, XP002242681, ISSN: 0021-9738, DOI: 10.1172/JCI11459

- **Beatrice M. Magnusson ET AL: "Molecular Size as the Main Determinant of Solute Maximum Flux Across the Skin", Journal of Investigative Dermatology, vol. 122, no. 4, 1 April 2004 (2004-04-01) , pages 993-999, XP055079241, ISSN: 0022-202X, DOI: 10.1111/j.0022-202X. 2004.22413.x**

**Description**

Technical Field

The present invention relates to a composition for external preparations that contains as an essential ingredient a fatty acid-based ionic liquid with a transcription factor decoy dissolved therein.

Background Art

A transcription factor means a protein that binds to a DNA upstream or downstream of a promoter to control the transcription of a gene; as representative examples, NF-κB, E2F, GATA-3, STAT-1, STAT-6, Ets, AP-1 and the like can be mentioned.

Next, a decoy means a bait; any substance having a structure mimicking something which it is to bind to, or act on, is called a decoy. As a transcription factor decoy, a double-stranded oligonucleotide having the same base sequence as the transcription factor binding region on the genomic DNA is mainly used (Patent Documents 1 to 3). In the co-presence of a transcription factor decoy consisting of such an oligonucleotide, some transcription factors bind to the transcription factor decoy, rather than binding to the binding region on the genomic DNA to which it is to bind. For this reason, transcription factors that bind to the genomic gene decrease, resulting in a decreased activity of the transcription factor. In this case, these oligonucleotides function as fakes (baits) of the real binding region on the genomic gene to bind to the transcription factor; therefore, they are called transcription factor decoys. It is known that by administering a transcription factor decoy, it is possible to reduce the activity of the target transcription factor to treat or prevent a disease caused by the transcription factor.

For example, when an NF-κB decoy is used as the above-described transcription factor decoy, the contribution of NF-κB can be suppressed, so that symptoms such as those in allergies, asthma, and rheumatism can be suppressed or mitigated. For this reason, a new anti-inflammatory ointment based on an NF-κB decoy is being developed; in particular, a study of its clinical application as a therapeutic drug for facial lesions in severe atopic dermatitis is ongoing (Non-patent Document 1).

However, a problem arises in which the molecular weight of a transcription factor decoy is usually about 10,000 or more, much exceeding the range of molecular weights allowing transdermal absorption. This is because the barrier function to separate the outer world and the inner environment of the living organism is very well developed in the skin, and hence the skin permeation of substances having molecular weights exceeding 1,000 is considerably suppressed. Therefore, it has been deemed difficult to obtain a therapeutic effect by external application except for erosive lesions where the skin barrier function has decreased considerably, such as eroded sites. In actual treatment, results just as expected have been obtained; a major problem has arisen concerning how to increase the transdermal absorbability of transcription factor decoys such as NF-κB decoys.

Hence, to increase the skin permeability of transcription factor decoys, various preparation formulations have been investigated. For example, there are disclosures of a preparation formulation of a mixture of an NF-κB decoy, soybean phospholipid and petrolatum (Patent Document 4), a water-soluble gel preparation of an NF-κB decoy and sodium alginate (Patent Document 5), and a preparation having an NF-κB decoy carried on nanoparticle surfaces (Patent Document 6). However, none of them are fully satisfactory; in particular, any nonaqueous preparation formulation wherein an NF-κB decoy is stably present has not been known.

EP 1 362 600 A1 discloses an ointment comprising NF-κB decoy dissolved in stearyl alcohol and petrolatum.

Against this background, there is demand for the development of a new nonaqueous dermal external preparation of a transcription factor decoy.

Patent Document 1: Japanese Patent Domestic Re-publication 96/035430
Patent Document 2: JP-3392143
Patent Document 3: WO95/11687
Patent Document 4: JP-A-2006-89475
Patent Document 5: JP-A-2007-137891
Patent Document 6: JP-A-2008-056611
Non-patent Document 1: Japanese Journal of Clinical Medicine No. 63, extra issue 12, p659-663 (2005)

Disclosure of the Invention

Problems to Be Solved by the Invention

The present invention is directed to providing an external preparation composition according to claim 1, wherein a transcription factor decoy such as an NF-κB decoy is stably dissolved to improve the transdermal absorbability thereof.

Means of Solving the Problems

Since some of the present inventors had discovered that giant nucleic acids like calf thymus DNA can be dissolved using an ionic liquid (Japanese Patent Application 2007-018004), the present inventors conceptualized utilizing an ionic liquid for a transcription factor decoy, conducted extensive investigations, and found that by using as a solvent a fatty acid-based ionic liquid having 2 to 20 carbon atoms under non-aqueous conditions, it is possible to increase the solubility of the transcription factor decoy in external preparations, and to improve the transdermal absorbability of the transcription factor decoy.

The present inventors also found that by blending plural fatty acid-based ionic liquids, it is possible to control the solubility of a transcription factor decoy in external preparations. Then, the present inventors found that by controlling the solubility, it is possible to promote the transdermal absorbability of a transcription factor decoy. Hence, the present inventors found that because the transdermal absorbability of a transcription factor decoy is improved by controlling the solubility thereof in the preparation of the present invention, the decoy is transdermally absorbed to exhibit its effect even in preparations containing the decoy at extremely low concentrations (0.0001 to 1w/w%) that have not been reported.

Based on these findings, the present inventors have developed the present invention.

Accordingly, the gist of the present invention is as follows:

[1] A nonaqueous external preparation composition containing a transcription factor decoy dissolved in a fatty acid-based ionic liquid obtained from a fatty acid having 2 to 20 carbon atoms and an organic amine compound having 4 to 12 carbon atoms, wherein the transcription factor decoy is a double-stranded oligonucleotide consisting of 14 to 35 base pairs and selected from the group consisting of NF-κB, E2F, GATA-3, STAT-1, STAT-6, Ets and AP-1.

[2] The external preparation composition described in [1] above, wherein the fatty acid having 2 to 20 carbon atoms is one or more selected from the group consisting of glycolic acid, methoxyacetic acid, levulinic acid, capric acid and isostearic acid.

[3] The external preparation composition described in [1] or [2] above, wherein the organic amine compound having 4 to 12 carbon atoms is one or more selected from the group consisting of diethanolamine, triethanolamine, diisopropanolamine and triisopropanolamine.

[4] The nonaqueous external preparation composition described in any one of [1] to [3] above, wherein the fatty acid-based ionic liquid is a mixed ionic liquid of plural fatty acid-based ionic liquids.

[5] The nonaqueous external preparation composition described in [4] above, wherein the mixed ionic liquid is a mixture of an ionic liquid (I) that is the diethanolamine salt or triethanolamine salt of a fatty acid having 2 to 5 carbon atoms and an ionic liquid (II) that is the diisopropanolamine salt or triisopropanolamine salt of a fatty acid having 2 to 20 carbon atoms.

[6] The nonaqueous external preparation composition described in [5] above, wherein the fatty acid having 2 to 5 carbon atoms of the ionic liquid (I) is selected from the group consisting of glycolic acid, methoxyacetic acid and levulinic acid.

[7] The nonaqueous external preparation composition described in [6] above, wherein the fatty acid having 2 to 5 carbon atoms of the ionic liquid (I) is levulinic acid.

[8] The nonaqueous external preparation composition described in any one of [5] to [7] above, wherein the ionic liquid (II) is the diisopropanolamine salt or triisopropanolamine salt of a fatty acid having 2 to 5 carbon atoms.

[9] The nonaqueous external preparation composition described in [8] above, wherein the fatty acid having 2 to 5 carbon atoms of the ionic liquid (II) is levulinic acid.

[10] The nonaqueous external preparation composition described in [8] or [9] above, wherein the ratio by weight of the ionic liquid (I) and the ionic liquid (II) ranges from 2:1 to 1:10.

(10a) The nonaqueous external preparation composition described in [8] or [9] above, wherein the ratio by weight of the ionic liquid (I) and the ionic liquid (II) ranges from 1:1 to 1:5.

[11] The nonaqueous external preparation composition described in any one of [5] to [7] above, wherein the ionic liquid (II) is the diisopropanolamine salt or triisopropanolamine salt of a fatty acid having 6 to 13 carbon atoms.

[12] The nonaqueous external preparation composition described in [11] above, wherein the fatty acid having 6 to 13 carbon atoms of the ionic liquid (II) is capric acid.

[13] The nonaqueous external preparation composition described in [11] or [12] above, wherein the ratio by weight of the ionic liquid (I) and the ionic liquid (II) ranges from 40:1 to 2:1.

(13a) The nonaqueous external preparation composition described in [11] or [12] above, wherein the ratio by weight of the ionic liquid (I) and the ionic liquid (II) ranges from 10:1 to 2:1.

[14] The nonaqueous external preparation composition described in any one of [5] to [7] above, wherein the ionic liquid (II) is the diisopropanolamine salt or triisopropanolamine salt of a fatty acid having 14 to 20 carbon atoms.

[15] The nonaqueous external preparation composition described in [14] above, wherein the fatty acid having 14 to 20 carbon atoms of the ionic liquid (II) is isostearic acid.

[16] The nonaqueous external preparation composition described in [14] or [15] above, wherein the ratio by weight of the ionic liquid (I) and the ionic liquid (II) ranges from 1:1 to 1:50.

(16a) The nonaqueous external preparation composition described in [14] or [15] above, wherein the ratio by weight of the ionic liquid (I) and the ionic liquid (II) ranges from 1:2 to 1:30.

[17] The nonaqueous external preparation composition described in [8] above, wherein an ionic liquid (IIa) that is the diisopropanolamine salt or triisopropanolamine salt of a fatty acid having 14 to 20 carbon atoms is further contained.

[18] The nonaqueous external preparation composition described in [17] above, wherein the fatty acid having 14 to 20 carbon atoms of the ionic liquid (IIa) is isostearic acid.

[19] The nonaqueous external preparation composition described in [17] or [18] above, wherein the ratio by weight of the ionic liquid (I) and the ionic liquids (II) and (IIa) ranges from 1:1 to 1:50.

(19a) The nonaqueous external preparation composition described in [17] or [18] above, wherein the ratio by weight of the ionic liquid (I) and the ionic liquids (II) and (IIa) ranges from 1:2 to 1:30.

[20] The nonaqueous external preparation composition described in any one of [1] to [19] above, wherein the concentration of the transcription factor decoy is 0.0001 to 1w/w%.

[21] The nonaqueous external preparation composition described in any one of [1] to [19] above, wherein the concentration of the transcription factor decoy is 0.0001 to 0.2w/w%.

[22] The nonaqueous external preparation composition described in any one of [1] to [21] above, wherein an ionic liquid that is the diethanolamine salt or triethanolamine salt of a fatty acid having 2 to 5 carbon atoms is contained at a concentration 3 to 3000 times the concentration of the transcription factor decoy.

[23] The nonaqueous external preparation composition described in any one of [1] to [21] above, wherein an ionic liquid that is the diethanolamine salt or triethanolamine salt of a fatty acid having 2 to 5 carbon atoms is contained at a concentration 3 to 1000 times the concentration of the transcription factor decoy.

[24] The nonaqueous external preparation composition described in [22] or [23] above, wherein a mixed ionic liquid of plural fatty acid-based ionic liquids is contained, the concentration of the mixed ionic liquid being 0.008 to 30w/w%.

[25] The nonaqueous external preparation composition described in any one of [1] to [24] above, wherein an organic solvent is added.

[26] The nonaqueous external preparation composition described in [25] above, wherein the organic solvent is one or more selected from the group consisting of diethyl sebacate, isopropyl myristate and propylene carbonate.

[27] The nonaqueous external preparation composition described in any one of [1] to [26] above, wherein a chelating agent is added.

[28] The nonaqueous external preparation composition described in [27] above, wherein the chelating agent is edetic acid disodium salt.

[29] The nonaqueous external preparation composition described in any one of [1] to [28] above, wherein the composition is in the form of an ointment with the addition of an ointment base.

[30] The nonaqueous external preparation composition described in [29] above, wherein the ointment base is a gelled hydrocarbon or white petrolatum.

[31] The nonaqueous external preparation composition described in any one of [1] to [30] above, wherein the transcription factor is selected from the group consisting of NF-κB, E2F, GATA-3, STAT-1, STAT-6, Ets and AP-1.

[32] The nonaqueous external preparation composition described in [31] above, wherein the transcription factor decoy is an NF-κB decoy comprising the sequence GGRHTYYHC (wherein R stands for A or G, Y for C or T, and H for A, C or T).

[33] The nonaqueous external preparation composition described in [32] above, wherein the NF-κB decoy comprises the sequence GGATTTCCC or GGACTTTCC.

[34] The nonaqueous external preparation composition described in [31] above, wherein the transcription factor decoy is an E2F decoy comprising the sequence TTTSSCGS (wherein S stands for G or C).

[35] The nonaqueous external preparation composition described in [34] above, wherein the E2F decoy comprises the sequence TTTCCCGC.

[36] The nonaqueous external preparation composition described in [31] above, wherein the transcription factor decoy is a GATA-3 decoy comprising the sequence WGATAR (wherein W stands for A or T, and R for A or G).

[37] The nonaqueous external preparation composition described in [36] above, wherein the GATA-3 decoy comprises the sequence AGATAG.

[38] The nonaqueous external preparation composition described in [31] above, wherein the transcription factor decoy is an STAT-1 decoy comprising the sequence TTCNNNGAA (wherein N stands for A, G, T or C).

[39] The nonaqueous external preparation composition described in [38] above, wherein the STAT-1 decoy comprises the sequence TTCCGGGAA.

[40] The nonaqueous external preparation composition described in [31] above, wherein the transcription factor decoy is an STAT-6 decoy comprising the sequence TTCNNNNGAA (wherein N stands for A, G, T or C).

[41] The nonaqueous external preparation composition described in [40] above, wherein the STAT-6 decoy comprises the sequence TTCCCAAGAA.

[42] The nonaqueous external preparation composition described in [31] above, wherein the transcription factor decoy is an Ets decoy comprising the sequence MGGAW (wherein M stands for A or C, and W for A or T).

[43] The nonaqueous external preparation composition described in [42] above, wherein the Ets decoy comprises the sequence CGGAA.

[44] The nonaqueous external preparation composition described in [31] above, wherein the transcription factor decoy is an AP-1 decoy comprising the sequence TGASTMA (wherein S stands for G or C, and M for A or C).

[45] The nonaqueous external preparation composition described in [44] above, wherein the AP-1 decoy comprises the sequence TGAGTCA.

[46] A method of suppressing gene transcription regulation by a transcription factor that binds to a transcription factor decoy contained in the nonaqueous external preparation composition described in any one of [1] to [45] above, comprising transdermally administering the nonaqueous external preparation composition to a mammal in need of suppressing the gene transcription regulation.

[47] A use of a transcription factor decoy and a fatty acid-based ionic liquid obtained from a fatty acid having 2 to 20 carbon atoms and an organic amine compound having 4 to 12 carbon atoms, for manufacturing a nonaqueous external preparation composition.

[48] The use described in [47] above, wherein the ionic liquid is a mixed ionic liquid of the ionic liquid (I) that is the diethanolamine salt or triethanolamine salt of a fatty acid having 2 to 5 carbon atoms and the ionic liquid (II) that is the diisopropanolamine salt or triisopropanolamine salt of a fatty acid having 2 to 20 carbon atoms.

Effect of the Invention

By preparing an external preparation composition with a transcription factor decoy according to claim 1 and an ionic liquid based on a fatty acid having 2 to 20 carbon atoms as essential ingredients, it is possible to stabilize the transcription factor decoy, and also to improve the transdermal absorbability of the transcription factor decoy. By using plural fatty acid-based ionic liquids, it is possible to control the solubility of the transcription factor decoy to further improve the transdermal absorbability. In particular, by preparing a nonaqueous external preparation composition, the stability of the transcription factor decoy can be improved. As stated above, it is possible to provide various external preparation products such as liquids, ointments, and patches, containing a transcription factor decoy using the present invention.

Brief Description of the Drawings

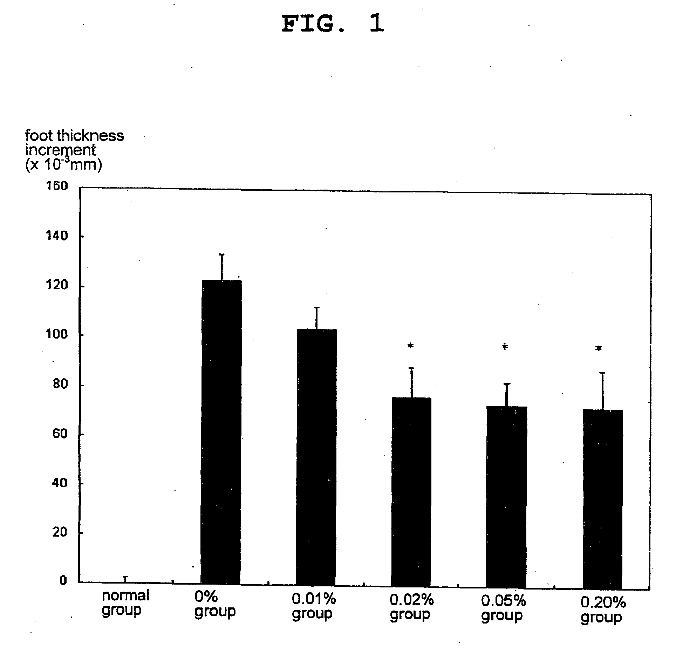

Fig. 1 is a graph showing evaluation results using a mouse DTH model. In the figure, * indicates $p < 0.05$ (vs. 0% group).

Best Mode for Carrying out the Invention

The present invention provides a composition for external preparations according to claim 1, that contains as essential ingredients a transcription factor decoy and an ionic liquid based on a fatty acid having 2 to 20 carbon atoms.

The transcription factor targeted by a decoy in the present invention is NF-κB, E2F, GATA-3, STAT-1, STAT-6, Ets, and AP-1.

"A transcription factor decoy" is a nucleic acid capable of binding to a transcription factor competitively with the region on the genomic DNA which the transcription factor essentially binds to, or acts on; the transcription factor decoy is a double-stranded oligonucleotide comprising the same or substantially the same base sequence as the transcription factor binding region on the genomic DNA, and consisting of 14 to 35 basepairs, more preferably 15 to 30 basepairs, still more preferably 17 to 25 basepairs, most preferably 18 to 22 basepairs. "Substantially the same base sequence" refers to a base sequence wherein 1, 2 or 3 bases are replaced with other bases in the consensus sequence of the transcription factor binding region, and which possesses bindability to the transcription factor equivalent to that of the consensus sequence or more (for example, 90% or more). The base sequence on the genomic DNA to which the desired transcription factor binds actually is widely variable, and a large number of cis-element sequences are known to be similar, but not completely identical, to the consensus sequence; therefore, those skilled in the art are able to easily design substantially the same base sequence on the basis of sequence information on a publicly known cis-element. Although the double-stranded oligonucleotide may be any of a double-stranded DNA, a double-stranded RNA, and a DNA:RNA hybrid, it is preferably a double-stranded DNA.

Base sequences found in common in transcription factor binding regions (cis-elements) (consensus sequences) are well known for respective transcription factors, each normally consisting of about 5 to 15 basepairs. As examples of consensus sequences to which representative transcription factors bind (sense strand sequences are shown; the same applies below), the following can be mentioned.

(1) Transcription factor NF-κB: sequence GGRHTYYHC (wherein R stands for A or G, Y for C or T, and H for A, C or T)
(2) Transcription factor E2F: sequence TTTSSCGS (wherein S stands for G or C)
(3) Transcription factor GATA-3: WGATAR (wherein W stands for A or T, and R for A or G)
(4) Transcription factor STAT-1: sequence TTCNNNGAA (wherein N stands for A, G, T or C)
(5) Transcription factor STAT-6: sequence TTCNNNNGAA (wherein N stands for A, G, T or C)
(6) Transcription factor Ets: sequence MGGAW (wherein M stands for A or C, and W for A or T)
(7) Transcription factor AP-1: sequence TGASTMA (wherein S stands for G or C, and M for A or C)

Disclosed are modified forms of double-stranded decoy oligonucleotides, dumbbell-shaped decoy oligonucleotides, dumbbell-shaped decoy oligonucleotides having an intramolecular nick, hairpin-shaped decoy oligonucleotides and the like.

As examples of more specific sequences homologous to cis-elements contained in decoys for the above-described transcription factors, the following can be mentioned, respectively.

(1a) NF-κB decoy: sequence GGATTTCCC or GGACTTTCC
(2a) E2F decoy: sequence TTTCCCGC
(3a) GATA-3 decoy: sequence AGATAG
(4a) STAT-1 decoy: sequence TTCCGGGAA
(5a) STAT-6 decoy: sequence TTCCCAAGAA
(6a) Ets decoy: sequence CGGAA
(7a) AP-1 decoy: sequence TGAGTCA

As stated above, the cis-element sequence to which a transcription factor binds usually consists of about 5 to 15 base pairs; therefore, a transcription factor decoy commonly used has a total of 1 to 30, preferably 1 to 25, more preferably 2 to 20, particularly preferably 3 to 17, basepairs added to one or both of the ends thereof. The base (sequence) to be added may be any one, as far as it does not adversely influence the properties of the transcription factor decoy.

More specifically, double-stranded oligonucleotides consisting of respective base sequences (target sequence for transcription factor is underlined) such as those shown below can be mentioned:

(1b) as NF-κB decoys, for example:

5'-CCTTGAAGGGATTTCCCTCC-3' (SEQ ID NO:1),
5'-AGTTGAGGGGACTTTCCCAGGC-3' (SEQ ID NO:2),
5'-AGTTGAGGACTTTCCAGGC-3' (SEQ ID NO:3) and the like,

(2b) as E2F decoys, for example:

5'-CTAGATTTCCCGCG-3' (SEQ ID NO:4),
5'-CTAGATTTCCCGCGGATC-3' (SEQ ID NO:5) and the like,

(3b) as GATA-3 decoys, for example:

5'-AGCTTGAGATAGAGCT-3' (SEQ ID NO:6) and the like,

(4b) as STAT-1 decoys, for example:

5'-GATCTAGGGATTTCCGGGAAATGAAGCT-3' (SEQ ID NO:7),
5'-TGTGAATTACCGGAAGTG-3' (SEQ ID NO:8) and the like,

(5b) as STAT-6 decoys, for example:

5'-GATCAAGACCTTTTCCCAAGAAATCTAT-3' (SEQ ID NO:9) and the like,

(6b) as Ets decoys, for example:

5'-AATTCAC<u>CGGAA</u>GTATTCGA-3' (SEQ ID NO:10) and the like,

(7b) as AP-1 decoys, for example:

5'-AGCTTG<u>TGAGTCA</u>GAAGCT-3' (SEQ ID NO:11) and the like.

As the oligonucleotide that constitutes the transcription factor decoy, natural type DNA or RNA, particularly DNA, is preferably used; to improve the stability (chemical and/or to enzyme) or specific activity (affinity for transcription factor), various chemical modifications can be contained.

Although a transcription factor decoy can be produced by, for example, synthesizing each of the sense strand and antisense strand of a single-stranded oligonucleotide consisting of a base sequence designed as described above, using a commercially available automated DNA/RNA synthesizer (Applied Biosystems, Beckman and the like), and annealing the two, another method publicly known in the art may be used.

The content of transcription factor decoy to be dissolved in the external preparation composition of the present invention may be such that the amount finally formulated in the external preparation results in delivery of a therapeutically effective level after transdermal absorption. The content of transcription factor decoy to be formulated in the external preparation composition may be, for example, 0.0001 to 1w/w%. More preferably, 0.0001 to 0.2w/w% can be mentioned. Most preferably, 0.0001 to 0.1w/w% can be mentioned.

In the external preparation composition of the present invention, the transcription factor decoy is dissolved in an ionic liquid based on a fatty acid having 2 to 20 carbon atoms.

Herein, "an ionic liquid based on a fatty acid having 2 to 20 carbon atoms" refers to an equimolar molten salt or equimolar equilibration mixture of a fatty acid having 2 to 20 carbon atoms and an organic amine compound having 4 to 12 carbon atoms; in consideration of the concentration of transcription factor decoy used and the influences of other additives, an excess amount of a fatty acid or organic amine may be added to the external preparation composition. The excess amount is desirably within a 0.2 fold molar amount.

As "fatty acids having 2 to 20 carbon atoms" in the present invention, medium-chain fatty acids having 2 to 10 carbon atoms, for example, glycolic acid, levulinic acid, caproic acid, octanoic acid, capric acid, and the like; saturated or unsaturated higher fatty acids having 11 to 20 carbon atoms, for example, lauric acid, myristic acid, palmitic acid, stearic acid, isostearic acid, oleic acid, and the like, and the like can be used. As examples of preferred ones, fatty acids having 5 to 20 carbon atoms, such as levulinic acid, capric acid, myristic acid, isostearic acid, and oleic acid, can be mentioned. Furthermore, an appropriate mixed ionic liquid may be prepared and used, in which the solubility of transcription factor decoy is controlled by appropriately using a plurality of these acids. As particularly preferred fatty acids, levulinic acid and isostearic acid can be mentioned.

In the present invention, "an organic amine compound having 4 to 12 carbon atoms" refers to an organic amine compound wherein the number of carbon atoms is 4 to 12. As preferable ones, linear or branched alkylamine compounds substituted by a hydroxyl group can be mentioned. As more preferable ones, diethanolamine, triethanolamine, diisopropanol amine, triisopropanolamine and the like can be mentioned.

The fatty acid-based ionic liquid used in the present invention may be a single ionic liquid or a mixed ionic liquid. Herein, "a mixed ionic liquid" refers to a mixture of plural the above-described ionic liquids based on a fatty acid having 2 to 20 carbon atoms; 3 kinds of mixed ionic liquids exist: ones having a common fatty acid and different organic amine compounds, ones having a common organic amine compound and different fatty acids, and ones having different fatty acids and different organic amine compounds. In the present invention, to control the solubility or transdermal absorbability of transcription factor decoy, suitable combinations can be used as appropriate. The content ratio of mixed ionic liquid can be determined in consideration of the role of ionic liquid.

To control the solubility of transcription factor decoy, a mixture of an ionic liquid offering high solubility and an ionic liquid offering low solubility is used.

As preferred ionic liquids in which the solubility of transcription factor decoy is high (ionic liquid for dissolution = ionic liquid (I)), ones wherein the decoy solubility is 10w/w% or more can be mentioned. As more preferable ones, ones wherein the decoy solubility is 20w/w% or more can be mentioned. As particularly preferable ones, ones wherein the decoy solubility is 28w/w% or more can be mentioned.

More specifically, as ionic liquids for dissolution offering high solubility of transcription factor decoy (ionic liquid (I)), the diethanolamine salts or triethanolamine salts of fatty acids having 2 to 5 carbon atoms can be mentioned. Here, as examples of fatty acids having 2 to 5 carbon atoms, glycolic acid, methoxyacetic acid, levulinic acid and the like can be mentioned. More preferably, levulinic acid can be mentioned. Therefore, as examples of ionic liquids for dissolution of transcription factor decoy, levulinic acid diethanolamine salt, levulinic acid triethanolamine salt, glycolic acid diethanolamine salt, glycolic acid triethanolamine salt, methoxyacetic acid diethanolamine salt, methoxyacetic acid triethanolamine salt and the like can be mentioned.

Meanwhile, as preferred ionic liquids in which the solubility of transcription factor decoy is low (ionic liquid for dilution = ionic liquid (II)), ones wherein the decoy solubility is 1w/w% or less can be mentioned. As more preferable ones, ones wherein the decoy solubility is 0.5w/w% or less can be mentioned. As particularly preferable ones, ones wherein the decoy solubility is 0.2% or less can be mentioned.

More specifically, as ionic liquids for dilution offering low solubility of transcription factor decoy (ionic liquid (II)), the diisopropanolamine salts or triisopropanolamine salts of fatty acids having 2 to 20 carbon atoms can be mentioned. Preferably, the diisopropanol amine salt or triisopropanolamine salt of a fatty acid having 5 to 20 carbon atoms can be mentioned. Here, as examples of fatty acids having 5 to 20 carbon atoms, levulinic acid, capric acid, myristic acid, isostearic acid, oleic acid and the like can be mentioned. More preferably, levulinic acid and isostearic acid can be mentioned. Therefore, as examples of ionic liquids offering low solubility of transcription factor decoy, levulinic acid diisopropanol amine salt, levulinic acid triisopropanolamine salt, capric acid diisopropanolamine salt, capric acid triisopropanolamine salt, isostearic acid diisopropanolamine salt, isostearic acid triisopropanolamine salt and the like can be mentioned. Preferably, levulinic acid diisopropanolamine salt, levulinic acid triisopropanolamine salt, isostearic acid diisopropanolamine salt, isostearic acid triisopropanolamine salt and the like can be mentioned.

The fatty acid-based ionic liquid of the present invention can be prepared by, for example, blending a fatty acid having 2 to 20 carbon atoms and an organic amine compound, preferably an organic amine compound having 4 to 12 carbon atoms, in an equimolar ratio or in an excess amount of either one as required, at room temperature or under warming. The excess amount is desirably within a 0.2 fold molar amount. A mixed ionic liquid can be obtained by, for example, blending ionic liquids prepared as described above in an appropriate content ratio.

Regarding the amount of ionic liquid for dissolving a transcription factor decoy, it is necessary to first dissolve the transcription factor decoy; therefore, it is first necessary to use an ionic liquid offering high solubility. It is important that after being dissolved, the decoy be diluted with an ionic liquid for dilution to make the solubility in the mixed ionic liquid closer to saturation. Therefore, as an amount of ionic liquid for decoy dissolution, it is necessary to set the amount at a level 3 to 3000 times higher than the amount of transcription factor decoy. As an appropriate amount of ionic liquid used, more preferably, an amount 3 to 1000 times, particularly preferably 3 to 800 times, higher than the amount of decoy, can be mentioned.

The concentration of the fatty acid-based ionic liquid in the external preparation composition of the present invention is largely influenced by the form of the external preparation; the concentration is preferably, for example, 10 to 50w/w% for liquids, for example, about 0.008 to 30w/w% for ointments. As more preferable concentrations in ointment preparations, 0.01 to 20w/w% can be mentioned; as most preferable concentrations, 0.01 to 10w/w% can be mentioned.

When a single ionic liquid is used, it is possible to use an ionic liquid for dissolution offering high solubility of transcription factor decoy alone. In that case, the ionic liquid concentration in the external preparation composition is also related to the amount of transcription factor decoy used; as the amount of transcription factor decoy used decreases, the relative amount of fatty acid-based ionic liquid used decreases. For example, when the levulinic acid triethanolamine salt or levulinic acid diethanolamine salt is used, it is desirable that the salt be added at a concentration about 3 to 3000 times higher than the decoy concentration. As preferable concentrations of single ionic liquid, concentrations 3 to 200 times higher than the decoy concentration can be mentioned. As particularly preferable concentrations of single ionic liquid, concentrations 3 to 100 times higher than the decoy concentration can be mentioned.

To adjust the decoy solubility in ionic liquid, a mixed ionic liquid comprising a blend of plural ionic liquids can be used. In that case, as ionic liquids for dissolution (ionic liquid (I)), one or more ionic liquids that are the diethanolamine salts or triethanolamine salts of fatty acids having 2 to 5 carbon atoms can be used. As ionic liquids for dilution (ionic liquid (II)), one or more ionic liquids that are the diisopropanolamine salts or triisopropanolamine salts of fatty acids having 2 to 20 carbon atoms can be used.

Regarding the content ratio of mixed ionic liquid, the preferred range varies depending on the choice of fatty acid of the ionic liquid for dilution (specifically, the fatty acid of the ionic liquid for dilution is (1) a fatty acid having 2 to 5 carbon atoms, (2) a fatty acid having 14 to 20 carbon atoms, or (3) a fatty acid having 6 to 13 carbon atoms).

In the present invention, as examples of "fatty acids having 2 to 5 carbon atoms", glycolic acid, methoxyacetic acid, ethoxyacetic acid, levulinic acid, lactic acid and the like can be mentioned; as preferable ones, glycolic acid, methoxyacetic acid, and levulinic acid can be mentioned. As a particularly preferable one, levulinic acid can be mentioned.

In the present invention, as examples of "fatty acids having 6 to 13 carbon atoms", 2-ethylhexanoic acid, capric acid and the like can be mentioned; as a preferable one, capric acid can be mentioned.

In the present invention, as examples of "fatty acids having 14 to 20 carbon atoms", myristic acid, isostearic acid, oleic acid and the like can be mentioned; as a preferable one, isostearic acid, oleic acid can be mentioned.

(1) When an ionic liquid for dissolution that is the diethanolamine salt or triethanolamine salt of a fatty acid having 2 to 5 carbon atoms (ionic liquid (I)) and an ionic liquid for dilution that is the triisopropanolamine salt or diisopropanolamine salt of a fatty acid having 2 to 5 carbon atoms (ionic liquid (II)) are used, a ratio by weight of the mixed ionic liquid in the range of ionic liquid (I):ionic liquid (II) = 2:1 to 1:10 can be mentioned. Preferably, a ratio of ionic liquid (I):ionic liquid (II) in the range of 1:1 to 1:5 can be mentioned. For example, when the levulinic acid triethanolamine salt is used as an ionic liquid for dissolution (ionic liquid (I)), and the levulinic acid triisopropanolamine salt as an ionic liquid for dilution (ionic liquid (II)), a ratio by weight of ionic liquid (I):ionic liquid (II) = 1:1 to 1:10 can be used. Preferably, ionic liquid (I):ionic liquid (II) = 1:1 to 1:5 can be mentioned. For example, when the levulinic acid diethanolamine salt is used as an ionic liquid for dissolution (ionic liquid (I)), and the levulinic acid triisopropanolamine salt as an ionic liquid for dilution (ionic liquid (II)), a ratio by weight of ionic liquid (I):ionic liquid (II) = 2:1 to 1:10 can be used. Preferably, ionic liquid (I):ionic liquid (II) = 1:1 to 1:4 can be mentioned. For example, when the glycolic acid triethanolamine salt is used as an ionic liquid for dissolution (ionic liquid (I)), and the glycolic acid triisopropanolamine salt as an ionic liquid for dilution (ionic liquid (II)), a ratio by weight of ionic liquid (I):ionic liquid (II) is preferably in the range of 1:1 to 1:8, more preferably 1:1 to 1:4.

(2) When an ionic liquid that is the diethanolamine salt or triethanolamine salt of a fatty acid having 2 to 5 carbon atoms is used as an ionic liquid for dissolution (ionic liquid (I)), and an ionic liquid that is the diisopropanolamine salt or triisopropanolamine salt of a fatty acid having 14 to 20 carbon atoms as an ionic liquid for dilution (ionic liquid (II)), a mixed ionic liquid wherein the content ratio by weight of the mixed ionic liquid is ionic liquid (I):ionic liquid (II) = 1:1 to 1:50 can be used. Preferably, a mixed ionic liquid of ionic liquid (I):ionic liquid (II) = 1:2 to 1:30 can be mentioned. More preferably, a mixed ionic liquid of ionic liquid (I):ionic liquid (II) = 1:2 to 1:20 can be mentioned. For example, when the levulinic acid triethanolamine salt is used as an ionic liquid for dissolution (ionic liquid (I)), and the isostearic acid diisopropanol amine salt as an ionic liquid for dilution (ionic liquid (II)), a ratio by weight of ionic liquid (I):ionic liquid (II) = 1:0 to 1:50 can be used. Preferably, a ratio by weight of ionic liquid (I):ionic liquid (II) = 1:2 to 1:30 can be mentioned. More preferably, a ratio by weight of ionic liquid (I):ionic liquid (II) = 1:2 to 1:20 can be mentioned.

(3) When an ionic liquid that is the diethanolamine salt or triethanolamine salt of a fatty acid having 2 to 5 carbon atoms is used as an ionic liquid for dissolution (ionic liquid (I)), and an ionic liquid that is the diisopropanolamine salt or triisopropanolamine salt of a fatty acid having 6 to 13 carbon atoms as an ionic liquid for dilution (ionic liquid (II)), a mixed ionic liquid wherein the ratio by weight of ionic liquid (I):ionic liquid (II) is in the range of 40:1 to 2:1 can be used. Preferably, a mixed ionic liquid wherein the ratio of ionic liquid (I):ionic liquid (II) is 10:1 to 2:1 can be mentioned. For example, when the levulinic acid triethanolamine salt is used as an ionic liquid for dissolution (ionic liquid (I)), and the capric acid diisopropanol amine salt as an ionic liquid for dilution (ionic liquid (II)), a ratio by weight of ionic liquid (I):ionic liquid (II) = 40:1 to 2:1 can be used. Preferably, a ratio by weight of ionic liquid (I):ionic liquid (II) = 10:1 to 2:1 can be mentioned.

It is also possible to blend 3 kinds or more of ionic liquids; for example, it is possible to use an ionic liquid that is the diethanolamine salt or triethanolamine salt of a fatty acid having 2 to 5 carbon atoms as an ionic liquid for dissolution (ionic liquid (I)), and an ionic liquid that is the triisopropanolamine salt or diisopropanolamine salt of a fatty acid having 2 to 5 carbon atoms (II), and an ionic liquid that is the diisopropanolamine salt or triisopropanolamine salt of a fatty acid having 14 to 20 carbon atoms (IIa) as ionic liquids for dilution. In this case, a mixed ionic liquid wherein the ratio by weight of ionic liquid (I):ionic liquid (II)+ionic liquid (IIa) is in the range of 1:1 to 1:50 can be used. Preferably, a mixed ionic liquid wherein the ratio by weight of ionic liquid (I):ionic liquid (II)+ionic liquid (IIa) is 1:2 to 1:30 can be mentioned. For example, combining the levulinic acid triethanolamine salt (ionic liquid (I)) with the levulinic acid triisopropanolamine salt (ionic liquid (II)) and the isostearic acid diisopropanolamine salt (ionic liquid (IIa)) as ionic liquids for dilution, a ratio by weight of ionic liquid (I):ionic liquid (II)+ionic liquid (IIa) = 1:2 to 1:30 can be used.

Because the content ratio of mixed ionic liquid is related to the decoy solubility in ionic liquid as described above, the decoy concentration is adjusted to a level as close to the decoy saturation concentration in the ionic liquid as possible, in consideration of the transcription factor decoy content (concentration) in the preparation. For example, when the transcription factor decoy concentration is 0.0001 to 0.5w/w%, the content ratio of ionic liquid for dissolution and ionic liquid for dilution can be adjusted with reference to the decoy solubility and the like shown in Example 2. For example, when the transcription factor decoy concentration in the preparation is 0.02%, the levulinic acid triethanolamine salt is used as an ionic liquid for dissolution (ionic liquid (I)), and the isostearic acid diisopropanolamine salt is used as an ionic liquid for dilution (ionic liquid (II)), the ratio by weight of the mixed ionic liquid is preferably in the range of 1:0 to 1:30 relative to the amount of the levulinic acid triethanolamine salt as 1. More preferably, a ratio by weight in the range of 1:2 to 1:20, still more preferably 1:2 to 1:13, can be mentioned.

Herein, the term "external preparation composition" is used as a concept encompassing both a nonaqueous external preparation composition and an aqueous external preparation composition. "A nonaqueous external preparation composition" is an external preparation composition that does not contain water as a constituent ingredient. In nonaqueous external preparation compositions, the absorbed or adsorbed water contained in the reagent is not taken into account. Meanwhile, "an aqueous external preparation composition" (not according to the invention) contains water as a constituent. The amount of water contained varies depending on the shape of the preparation, and is desirably an amount that allows water to be well mixed with the base and uniformly dispersed. As a water concentration in the preparation, about 10% or less is preferred.

The nonaqueous external preparation composition of the present invention can contain an organic solvent in addition to the above-described composition for external preparations.

Herein, "an organic solvent" refers to a solvent that functions to dilute and solvate a transcription factor decoy in a fatty acid-based ionic liquid. Furthermore, ones that act on the skin surface to improve the transdermal absorbability are desirable. For example, ethers, such as THF, butyl ether, polyethylene glycol methyl ether; ketones, such as methyl isobutyl ketone; lower alkyl carboxylic acid esters, such as ethyl acetate, propyl acetate, ethyl butyrate; fatty acid esters, such as diethyl sebacate, isopropyl myristate, diisopropyl adipate, myristyl palmitate, stearyl stearate, myristyl myristate, oleic triglyceride, ceryl lignocerate, lacceryl cerophosphate, lacceryl laccerate; carbonic acid esters, such as propylene carbonate; vegetable oils, such as olive oil and coconut oil, and the like can be mentioned. As preferable ones, fatty acid esters such as isopropyl myristate and diethyl sebacate, vegetable oils such as coconut oil and olive oil can be mentioned. Furthermore, higher alcohols, such as benzyl alcohol, lauryl alcohol, myristyl alcohol, cetyl alcohol, stearyl alcohol, cetostearyl alcohol, 2-octyldodecanol; lower alcohols having 1 to 12 carbon atoms, such as ethanol, propanol, isopropanol, n-butanol, pentanol, octanol, and dodecanol; polyhydric alcohols, such as ethylene glycol, glycerin, propylene glycol, and 1,3-butylene alcohol, and the like can be mentioned. As preferred ones, ethanol, isopropanol, ethylene glycol, and propylene glycol can be mentioned.

Presence or absence of addition of these organic solvents, and an amount added can be chosen as appropriate if required. Preferably, a range of 0.5 to 10w/w% can be mentioned.

The nonaqueous external preparation composition of the present invention can further contain a chelating agent. Herein, "a chelating agent" refers to a chelating agent used to remove divalent metal ions such as Zn so as to suppress the activity of DNAases present on the skin surface. Specifically, ones that can be used for this purpose with low dermal irritability can be mentioned; for example, edetic acid disodium salt (EDTA•2Na), acetylacetic acid esters such as acetylacetic acid methyl ester and acetylacetic acid ethyl ester, and the like can be mentioned. Preferably, EDTA•2Na can be mentioned.

Herein, "an external preparation" can be prepared in the form of various dosage forms of external preparations according to the target disease and the intended use. For example, liquids, gels, ointments, creams, lotions, patches and the like can be mentioned. They can be produced using means in common use for pharmaceutical making.

Furthermore, as other additives, publicly known antioxidants, antiseptics, thickeners, and preservatives in common use can be used as appropriate.

As antioxidants, ascorbic acid, sodium hydrogen sulfite, sodium sulfite, erythorbic acid, tocopherol acetate, dibutylhydroxytoluene, tocopherol, sodium metabisulfite, butylhydroxyanisole, propyl gallate and the like can be mentioned.

As examples of antiseptics, benzoic acid, sodium benzoate, sorbic acid, sodium sorbate, sodium dehydroacetate, para-oxybenzoic acid, sodium para-oxybenzoate, ethyl para-oxybenzoate, propyl para-oxybenzoate (propylparaben), butyl para-oxybenzoate, isopropyl para-oxybenzoate, isobutyl para-oxybenzoate, propionic acid, sodium propionate, benzalkonium chloride, salicylic acid, mixtures thereof and the like can be mentioned. Preferably, methylparaben, propylparaben, benzalkonium chloride, salicylic acid, mixtures thereof and the like can be mentioned.

A thickener refers to a substance that is solid and sparingly soluble in water at normal temperature. As examples of inorganic materials, for example, amorphous silicon dioxide, kaolin (gypsum), diatomaceous earth, talc, hydrated silicon dioxide, light anhydrous silicic acid, magnesium silicate, calcium silicate, calcium phosphate, barium sulfate and the like can be mentioned. As examples of organic materials, crystalline cellulose and the like can be mentioned. As a preferred one, light silicic anhydride can be mentioned.

The external preparation composition of the present invention can be prepared by dissolving or suspending a pharmacologically effective ingredient in a base constituent ingredient for the preparation. As bases for external preparation compositions in the form of ointments, for example, white petrolatum, liquid paraffin, gelled hydrocarbon and the like can be mentioned. Gelled hydrocarbon is prepared by gelling a hydrocarbon such as liquid paraffin, paraffin, isoparaffin, squalane, squalene, polybutene or the like. Particularly, liquid paraffin gelled with polyethylene resin and an oil or fat gelled with rubber/elastomer are preferred. For example, Plastibase (trade name) or Poloid (trade name), which is prepared by gelling liquid paraffin (Japanese Pharmacopoeia) with 5 to 10% by weight of polyethylene resin, a hydrophilic gelled hydrocarbon prepared by adding a glycerin fatty acid ester to a gelled hydrocarbon to confer hydrophilicity (Plastibase Hydrophilic (trade name)) and the like can be mentioned.

In creams, a base prepared with, for example, an emulsion of a mixture of an oil ingredient such as squalane, liquid paraffin, petrolatum, lanolin, solid paraffin, or beeswax, water, a surfactant, a moisturizer and the like can be mentioned.

In liquids, a base prepared with a mixed solution of an alcohol such as isopropanol, ethanol, propylene glycol, or glycerin, an oil or fat such as olive oil or soybean oil, and water can be mentioned.

In patches, an adhesive agent is used as a base. As mentioned here, an adhesive agent mainly comprises an elastomer, tackifier, softening agent, filler, antioxidant and the like. In particular, the softening agent, filler, and antioxidant can be increased, decreased, or removed as appropriate if required.

As the above-described elastomer, synthetic rubbers such as styrene-isoprene-styrene block (hereinafter referred to as SIS) copolymer, styrene-butadiene-styrene block copolymer, styrene-ethylene-butadiene rubber-styrene block copolymer, styrene-butadiene rubber, polyisoprene, polyisobutylene, polybutene, butyl rubber, silicone rubber; acrylate resins such as polymethyl acrylate and polymethyl methacrylate; natural rubber and the like can be mentioned. As preferred ones, ones based on rubber polymers such as styrene-isoprene-styrene block copolymer, styrene-butadiene rubber, polybutene, polyisoprene, butyl rubber, and natural rubber can be mentioned. These may be used singly, or in combination of 2 kinds or more. The above-described resin films may be used singly, or in lamination of 2 kinds or more.

The above-described tackifier refers to an alicyclic hydrocarbon resin, a polyterpene resin, an aliphatic hydrocarbon resin, a polystyrene resin, a rosin, a hydrogenated rosin or the like. As a preferable one, an alicyclic hydrocarbon resin can be mentioned.

As examples of the above-described softening agent, petroleum-based softening agents such as processed oil and low-molecular polybutene, fatty oil-based softening agents such as castor oil and coconut oil, purified lanolin and the like can be mentioned.

As examples of the above-described fillers, those of zinc oxide, titanium oxide, calcium carbonate, silicic acids and the like can be mentioned.

Various transcription factors that are active ingredients of the external preparation composition of the present invention induce the expression of various inflammation-related genes, including cytokines, chemokines, growth factors, adhesion molecules, and apoptosis-related molecules. In particular, since NF-$\kappa$B induces the expression of many inflammation-related genes, NF-$\kappa$B decoys are expected to exhibit multiple anti-inflammatory actions by suppressing the expression of such genes, and to produce lower incidences of adverse reactions than steroids, because of the action specificity thereof. Currently, clinical applications for atopic dermatitis using this decoy are being investigated.

The external preparation composition of the present invention produced as described above is of low toxicity, and can be transdermally administered to a human or another mammal (e.g., mouse, rat, rabbit, sheep, pig, cattle, cat, dog, monkey and the like) in need of suppression of gene transcriptional control by a transcription factor that binds to a transcription factor decoy contained in the external preparation composition, for example, an animal suffering a disease for which the condition is expected to be ameliorated by suppressing the transcriptional regulation.

The dosing protocol for the external preparation composition of the present invention varies depending on the subject of administration, target disease, symptoms and the like; for example, for use to treat adult atopic dermatitis, an appropriate amount of the active ingredient NF-$\kappa$B decoy (as a single-dose amount, usually about 0.1 mg to 1 g, preferably about 1 to 500 mg) can be transdermally administered about 1 to 5 times a day, preferably about 1 to 3 times a day. If the symptom is particularly severe, the dose may be increased depending on the symptom.

Examples

The present invention is hereinafter described more specifically on the basis of the following Examples and Test Examples, by which, however, the present invention is not limited in any way. For NF-$\kappa$B decoys in ointment preparations, (1) a skin permeability test and (2) a mouse transdermal absorbability evaluation test with an OVA-induced mouse DTH model (model of delayed allergic reactions) were performed as described below.

(1) Skin permeability evaluation test of NF-$\kappa$B decoy (peeling test)

Based on the method specified in the "Guideline for Bioequivalence Studies of Generic Products of Preparations for Topical Skin Application" issued by the Ministry of Health, Labor and Welfare, with some modifications, the skin permeability of decoy was evaluated.

(a) Qualitative evaluation test:

An ointment preparation was applied to a range of 1 cm radius in the anterior surface of the subject's forearm; after elapse of 1 hour, the ointment preparation on the application site was carefully wiped off, and adhesive tapes were repeatedly applied and removed.

The first two of the sample tapes were discarded; a set of five tapes after the third tape were taken for one layer of the skin (for one test tube), and the tapes separately for the first to 3rd layers of the skin were acquired. An extraction solvent (3 ml of methanol and 0.5 ml of 10 mM Tris-EDTA (pH 7.0)) had previously been placed in the test tube, and each tape removed was immediately immersed in the extraction solvent. After completion of peeling, each tape was shredded and sonicated. The extract was filtered, and the solvent was distilled off to a residual amount of 0.2 ml using an evaporator.

100 $\mu$l of the concentrate solution obtained was collected and examined by HPLC for the presence or absence of decoy.

(b) Quantitative evaluation test:

An ointment preparation was applied to a range of 15 mm radius in the anterior surface of the subject's forearm; after elapse of 30 minutes, the ointment preparation on the application site was carefully wiped off, and adhesive tapes were repeatedly applied and removed. In the same manner as (1), tapes for the first to 3rd layers of the skin were acquired. An extraction solvent (2 ml of ultrapure water and 0.5 ml of 10 mM Tris-EDTA (pH 7.0)) had previously been placed in the test tube, and each tape removed was immediately immersed in the extraction solvent. After completion of peeling, each tape was shredded and sonicated. The extract was filtered, and the solvent was distilled off to a residual amount of 0.15 ml using an evaporator.

100 $\mu$l of the concentrate solution obtained was collected, and the amount of decoy collected was measured by HPLC. The amount of decoy detected in the tapes for the 3rd layer of the skin was used as a scale for skin permeability (ng/layer).

(2) Mouse transdermal absorbability evaluation test of NF-$\kappa$B decoy using OVA-induced mouse DTH model (model of delayed allergic reactions)

(1) Preparation of OVA and primary sensitization

A specified amount of ovalbumin (OVA) was weighed out, and an OVA solution 5 mg/mL was prepared with D-PBS [Dulbecco's Phosphate-buffered Salines] (-). An equal volume of Freund's incomplete adjuvant was added to an OVA 10 mg/mL solution, and the mixture was shaken using a Vortex (trade name) mixer for 5 minutes to form an emulsion, whereby an OVA/emulsion was prepared.

The OVA/emulsion was administered to BALB/c mice (female, 7 weeks of age) at two intradermal sites in the upper and lower parts of the abdomen at 100 $\mu$L/site.

(2) Administration of decoy

20 mg of each preparation was applied to the right foot 24 hours before secondary sensitization in case of single-dose administration, and 1, 2 and 3 days before secondary sensitization in case of repeated administration; 24 hours later, the drug was removed using absorbent cotton moistened with lukewarm water.

(3) Secondary sensitization

Nine days after primary sensitization, secondary sensitization was performed. A specified amount of OVA was weighed out, and an OVA 2 mg/mL solution was prepared with D-PBS(-). The mouse right foot was sensitized, and the left foot was left non-sensitized. The OVA 2 mg/mL was administered subcutaneously to the center of the back of the mouse sensitized foot at 50 $\mu$L/site. D-PBS(-) was administered to the non-sensitized foot at 50 $\mu$L/site.

(4) Measurement of DTH reactions

24 hours after secondary sensitization, foot thickness was measured in the center of each foot using a thickness gauge (produced by Mitutoyo). Three measurements were taken, and the mean was obtained as the foot thickness.

An evaluation was made by calculating increments of foot thickness by the equation:

$$\text{(Sensitized foot thickness)} - \text{(non-sensitized foot thickness)} = \text{(foot thickness increment)}$$

Individuals exhibiting internal hemorrhage in a foot due to an influence of administration at the time of secondary sensitization were excluded from the evaluation.

With the foot thickness increment suppression rate (%) in the test preparation dosing group relative to the foot thickness increment in the decoy-free preparation dosing group (0% group) as an index, transdermal absorbability (delayed allergic reaction suppression) was evaluated.

Intergroup comparisons were made by t-test.

Example 1: Nonaqueous liquid compositions of NF-$\kappa$B decoy

An NF-$\kappa$B decoy is a compound readily soluble in water, and it has been difficult to prepare a nonaqueous external preparation therewith. However, an NF-κB decoy could be dissolved in the following fatty acid-based ionic liquids to successfully liquefy the NF-κB decoy.

(1) Nonaqueous external preparation composition with levulinic acid-based ionic liquids (single ionic liquids):

Levulinic acid and an equimolar amount of each of diethanolamine and triethanolamine were weighed out and blended, and each resulting mixture was warmed at 80°C for 20 minutes to yield 2 kinds of levulinic acid-based ionic liquids.

About 10 mg of the NF-κB decoy shown by SEQ ID NO:1 was weighed out, and added to about 1.0 g of each of the 2 kinds of levulinic acid-based ionic liquids, and the mixture was warmed to at 80°C for about 30 minutes. After the mixture was allowed to cool to room temperature, the state of decoy dissolution was checked. As a result, the NF-κB decoy dissolved in each of the levulinic acid-based ionic acids of the diethanolamine salt and triethanolamine salt; nonaqueous liquids were successfully prepared.

Furthermore, the solubilities of the NF-κB decoy in the above-described 2 kinds of ionic liquids were determined; it was found that the solubilities are as shown in Table 1 below.

[Table 1]

| Levulinic acid-based ionic liquid | Amount of decoy | Amount of ionic liquid | Dissolution status | Solubility |
|---|---|---|---|---|
| Diethanolamine salt | 102.4 mg →119.1 mg | 70.6 mg | Soluble→ insoluble | 59.2 w/w% |
| Triethanolamine salt | 101.9 mg | 223.1 mg →258.3 mg | Insoluble→ soluble | 28.3 w/w% |

As shown in Table 1, the NF-κB decoy shown by SEQ ID NO:1 was found to be well soluble in ionic liquids of the levulinic acid diethanolamine salt or triethanolamine salt.

Meanwhile, the solubilities of the NF-κB decoy in the diisopropanol amine salt and triisopropanolamine salt of levulinic acid were determined in the same manner; the results shown in Table 2 below were obtained.

[Table 2]

| Levulinic acid-based ionic liquid | Amount of decoy | Amount of ionic liquid | Dissolution status | Solubility |
|---|---|---|---|---|
| Diisopropanolamine salt | 10.2 mg | 4.03 g →5.02 g | Insoluble→ soluble | 0.2 w/w% |
| Triisopropanolamine salt | 1.3 mg | 3.13 g →4.13 g | Insoluble→ soluble | 0.03 w/w% |

(2) Nonaqueous external preparation compositions with glycolic acid-based ionic liquids:

Glycolic acid and an equimolar amount of each of diethanolamine and triethanolamine were weighed out and blended together, and each resulting mixture was warmed at 80°C for 20 minutes to yield 2 kinds of glycolic acid-based ionic liquids.

10 mg of the NF-κB decoy shown by SEQ ID NO:1 was weighed out and dissolved in about 100 mg of each of ionic liquids of the glycolic acid diethanolamine salt and glycolic acid triethanolamine salt. The decoy dissolved in each of the ionic liquids to produce uniform solutions, whereby nonaqueous liquids were successfully prepared.

Example 2: Nonaqueous liquid compositions of NF-κB decoy (mixed ionic liquids)

Since the transdermal absorbability of external preparation is influenced by drug concentration and drug environment (whether or not close to saturation etc.), NF-κB decoy liquids of mixed ionic liquid solutions based mainly on levulinic acid were prepared as described below. Also, the solubilities of NF-κB decoy in the mixed ionic liquids were determined.

(1) Solubilities of NF-κB decoy in levulinic acid-based mixed ionic liquids (i):

Mixed ionic liquids were prepared wherein the levulinic acid triisopropanolamine salt was added in a ratio by weight of 1 to 9 relative to the amount of the levulinic acid triethanolamine salt as 1. The solubilities of the NF-κB decoy in these mixed ionic liquids were measured according to the method of Example 1. The results are shown in Tables 3-1 and 3-2 below.

[Table 3-1]

| Levulinic acid/trietha:levulinic acid/triiso (ratio by weight) | 0:10 | 1:9 | 2:8 | 1:3 2.5/7.5 | 3:7 | 5:5 | 10:0 |
|---|---|---|---|---|---|---|---|
| Decoy solubility (concentration w/w%) | 0.03 | 0.4 | 0.6 | 2 | 15 | 25 | 28.3 |
| [Note] •trietha: triethanolamine •triiso: triisopropanolamine | | | | | | | |

[Table 3-2]

| Levulinic acid/trietha: levulinic acid/triiso | 0:10 | 1:9 | 2:8 | 1:3 | 1:2.5 | 3: 7 | 1:2 | 5:5 | 10:0 |
|---|---|---|---|---|---|---|---|---|---|
| Decoy solubility (concentration w/w%) | <0.03 | 0.4 | 0.6 | 2.1 | 1.4 | 4.6 | 4.9 | 24.3 | 28.3 |
| [Note] •trietha: triethanolamine •triiso: triisopropanolamine | | | | | | | | | |

From these results, it was shown that the solubility of the NF-$\kappa$B decoy decreased greatly with the increase in the amount of an ionic liquid offering low solubility of decoy (levulinic acid triisopropanolamine salt) added relative to the amount of an ionic liquid offering high solubility of decoy (levulinic acid triethanolamine salt). Specifically, as shown in Tables 3-1 and 3-2, it was found that when the ratio of the ionic liquid offering low solubility of decoy to the ionic liquid offering high solubility of decoy is greater than about 1:2, the decoy solubility decreases rapidly.

(2) Solubilities of NF-$\kappa$B decoy in levulinic acid-based mixed ionic liquids (ii):

Mixed ionic liquids were prepared wherein the levulinic acid diisopropanol amine salt was added in a ratio by weight of 1 to 20 relative to the amount of the levulinic acid triethanolamine salt as 1, and the solubilities of the NF-$\kappa$B decoy in these mixed ionic liquids were measured according to the method of Example 1. The results are shown in Table 4 below.

[Table 4]

| Levulinic acid/trietha: levulinic acid/diiso | 0:1 | 1:20 | 1:16 | 1:13 | 1:10 | 1:4.6 | 1:2 | 1:1 | 1:0 |
|---|---|---|---|---|---|---|---|---|---|
| Decoy solubility (concentration w/w%) | 0.2 | 0.2 | 0.2 | 0.2 | 0.5 | 3.3 | 3.2 | 7.4 | 28.3 |
| [Note] •trietha: triethanolamine •triiso: triisopropanolamine | | | | | | | | | |

From these results, it was found that the levulinic acid diisopropanol salt lowers the decoy solubility more rapidly than by the addition of the levulinic acid triisopropanolamine salt.

(3) Solubilities of NF-$\kappa$B decoy in levulinic acid-based mixed ionic liquids (iii):

Mixed ionic liquids were prepared wherein the levulinic acid triisopropanolamine salt was added in a ratio by weight of 1 to 4 relative to the amount of the levulinic acid diethanolamine salt as 1, and the solubilities of the NF-$\kappa$B decoy in these mixed ionic liquids were measured according to the method of Example 1. The results are shown in Table 5 below.

[Table 5]

| Levulinic acid/dietha:levulinic acid/triiso | 0:1 | 1:4 | 1:3 | 1:1 | 2:1 | 1:0 |
|---|---|---|---|---|---|---|

(continued)

| Levulinic acid/dietha:levulinic acid/triiso | 0:1 | 1:4 | 1:3 | 1:1 | 2:1 | 1:0 |
|---|---|---|---|---|---|---|
| Decoy solubility (concentration w/w%) | <0.5 | 1.0 | 1.0 | 20.4 | 24.8 | 59.2 |
| [Note]<br>•dietha: diethanolamine<br>•triiso: triisopropanolamine | | | | | | |

From these results, it was found that the levulinic acid diethanol salt offers high solubility of decoy, and that when the levulinic acid triisopropanolamine salt was added in an amount 2 to 3 times higher, the solubility decreases significantly.

(4) Solubilities of NF-$\kappa$B decoy in mixed ionic liquids of levulinic acid-based ionic liquid and capric acid-based ionic liquid:

Mixed ionic liquids were prepared wherein the capric acid diisopropanol amine salt was added in a ratio by weight of 0.025 to 1 relative to the amount of the levulinic acid triethanolamine salt as 1, and the solubilities of the NF-$\kappa$B decoy in these mixed ionic liquids were measured according to the method of Example 1. The results are shown in Table 6 below.

[Table 6]

| Levulinic acid/trietha:capric acid/diiso | 1:1 | 2:1 | 4:1 | 6:1 | 8:1 | 10:1 | 20:1 | 40:1 | 1:0 |
|---|---|---|---|---|---|---|---|---|---|
| Decoy solubility (concentration w/w%) | <0.5 | <0.5 | 5.8 | 8.1 | 8.9 | 8.9 | 18.2 | 23.7 | 28.3 |
| [Note]<br>•trietha: triethanolamine<br>•diiso: diisopropanolamine | | | | | | | | | |

From these results, it was found that the capric acid diisopropanol salt rapidly lowers decoy solubility when added only in small amounts.

(5) Solubilities of NF-$\kappa$B decoy in glycolic acid-based mixed ionic liquids:

Mixed ionic liquids were prepared wherein the glycolic acid triisopropanolamine salt was added in a ratio by weight of 1 to 8 relative to the amount of the glycolic acid triethanolamine salt as 1, and the solubilities of the NF-$\kappa$B decoy in these mixed ionic liquids were measured according to the method of Example 1. The results are shown in Table 7 below.

[Table 7]

| Glycolic acid/trietha:glyclolic acid/triiiso | 0:1 | 1:8 | 1:4 | 1:2 | 1:1 | 4:1 | 1:0 |
|---|---|---|---|---|---|---|---|
| Decoy solubility (concentration w/w%) | <0.5 | <0.5 | <0.5 | 0.74 | 2.92 | 10.6 | 15 |
| [Note]<br>•trietha: triethanolamine<br>•triiso: triisopropanolamine | | | | | | | |

From these results, it was found that the decoy solubility decreases rapidly when the glycolic acid triisopropanol salt is added in an amount about 1 time higher to the glycolic acid triethanolamine salt.

Example 3: Ointment preparations of NF-$\kappa$B decoy

Ionic liquid solutions with a decoy dissolved therein were blended with a base for external preparations to investigate the preparation of various external preparation compositions. First, the decoy was blended with the ointment base to yield ointment preparations of the decoy, and the effects of ionic liquid on the skin permeability of the decoy were checked.

(1) Effects of levulinic acid-based ionic liquid in ointment preparations:

Levulinic acid and triethanolamine were weighed out and blended together in an equimolar ratio. An appropriate amount of propyl gallate was added thereto, and the mixture was warmed at 80°C for 20 minutes. The viscous solution obtained was weighed out at room temperature, the NF-κB decoy shown by SEQ ID NO:1 was added thereto, the decoy was thermally dissolved under 40°C conditions for 2 days, to yield a levulinic acid triethanolamine-based ionic liquid solution with the NF-κB decoy dissolved therein.

The ionic liquid solution was blended with separately weighed gelled hydrocarbon, light anhydrous silicic acid, methyl para-oxybenzoate, and disodium edetate. Furthermore, a mixed solution prepared by blending isostearic acid and diisopropanolamine under warming at 80°C for 20 minutes and an appropriate amount of propylene carbonate were added, whereby a total of 50.00 g of decoy ointment preparation was prepared.

To prepare an ointment preparation without using an ionic liquid for a comparative example, propylene carbonate, light anhydrous silicic acid, Plastibase (trade name) and the like were added to 250 mg of the NF-κB decoy to obtain the respective content ratios (w/w%) in Table 8 below to prepare an ointment preparation containing the NF-κB decoy. For these ointment preparations, skin permeability and mouse transdermal absorbability (repeated administration) were evaluated; the results are shown in Table 8.

[Table 8]

| Test number | Comparative Example 1 | Preparation Example 1 |
|---|---|---|
| NF-κB decoy | 0.05 | 0.05 |
| Ionic liquid: levulinic acid/trietha: | 0 | 0.4 |
| Solvent: propylene carbonate | 1.0 | 1.0 |
| Chelating agent: edetic acid 2Na | 0.8 | 0.8 |
| Antioxidant: propyl gallate | 0.2 | 0.2 |
| Antiseptic: methylparaben etc. | 0.06 | 0.2 |
| Thickener: light anhydrous silicic acid | 1.0 | 1.0 |
| Ointment base: Plastibase (trade name) | 96.89 | 96.35 |
| Skin permeability: qualitative evaluation test (peeling test 3rd layer) | N.D | + |
| Mouse transdermal absorbability test (delayed allergic reaction suppression rate) | - | 13.7% |
| [Note] •trietha: triethanolamine •N.D.: Not Detected •+: Detected •-: Not examined | | |

As shown in Table 8 above, in the absence of an ionic liquid, skin permeability of the NF-κB decoy was not confirmed. Meanwhile, in the presence of an ionic liquid, skin permeability of the NF-κB decoy was confirmed. In terms of the appearance of preparation, the NF-κB decoy was easily dissolved and dispersed in the ointment preparation in the presence of the ionic liquid, whereas in the absence of the ionic liquid, the NF-κB decoy did not dissolve but dispersed in the ointment preparation in a crystalline state.

(2) Influences of decoy solubility in ionic liquid (adjustment of decoy solubility with mixed ionic liquid):

a) Effects of levulinic acid-based mixed ionic liquids (base-mixed systems):

The decoy solubility in ionic liquid was adjusted using a mixed ionic liquid of the levulinic acid triethanolamine salt and levulinic acid triisopropanol amine salt. The ointment preparations shown by the content ratios (w/w%) in Table 9 below were prepared using 25 mg of the NF-κB decoy shown by SEQ ID NO:1 in the same manner as Example 3. For these ointment preparations, skin permeability and mouse transdermal absorbability (single-dose administration) were evaluated; the results are shown in Table 9.

[Table 9]

| Test number | Preparation Example 1 | Preparation Example 2 | Preparation Example 11 |
|---|---|---|---|
| NF-κB decoy | 0.05 | 0.05 | 0.05 |
| Ionic liquid: | levulinic acid/trietha 0.4 | levulinic acid/trietha: 0.925 levulinic acid/triiso: 2.775 | levulinic acid/trietha: 0.925 levulinic acid/triiso: 2.775 |
| Solvent: propylene carbonate | 1.0 | 1.0 | 0 |
| Chelating agent: edetic acid 2Na | 0.8 | 0.8 | 0.5 |
| Antioxidant: propyl gallate | 0.2 | 0.2 | Sodium hydrogen sulfite: 0.001 propyl gallate: 0.01 |
| Antiseptic: methylparaben etc. | 0.2 | 0.2 | 0.06 |
| Thickener: light anhydrous silicic acid | 1.0 | 1.0 | 0.25 |
| Ointment base: Plastibase (trade name) | 96.35 | 93.05 | 94.43 |
| Skin permeability: Qualitative evaluation test Quantitative evaluation test | + | + | + 1.0 (ng/layer) |
| Mouse transdermal absorbability test (delayed allergic reaction suppression rate) | 13.7% | - | 34.1%* |
| [Note] •trietha: triethanolamine •triiso: triisopropanolamine •+: Detected •*: p<0.05 (v. 0% group) | | | |

As shown by the results in Table 9, it was found that the ointment preparations prepared using a levulinic acid-based mixed ionic liquid gave better skin permeability than the ointment preparation prepared using a single ionic liquid of levulinic acid trietha.
Hence, from the results in Table 9, it was shown that the ointment preparations having a composition wherein the decoy concentration in the ionic liquid is closer to saturation gave better skin permeability.

b) Effects of mixed ionic liquid of levulinic acid-based ionic liquid and isostearic acid-based ionic liquid:

To adjust the solubility of the NF-κB decoy in ionic liquids, ointment preparations based on a mixed ionic liquid having the content ratios (w/w%) in Table 10 below were prepared.

The skin permeabilities (peeling test) and mouse transdermal absorbabilities (single-dose administration) of these ointment preparations are also shown in Table 10.

[Table 10]

| Test number | Preparation Example 1 | Preparation Example 3 | Preparation Example 4 |
|---|---|---|---|
| NF-κB decoy | 0.05 | 0.05 | 0.05 |
| Ionic liquid: | levulinic acid/trietha 0.4 | levulinic acid/trietha: 0.4 isostearic acid/diiso: 8.8 | levulinic acid/trietha: 0.8 isostearic acid/diiso: 3.67 |
| Solvent: propylene carbonate | 1.0 | 1.0 | 1.0 |
| Chelating agent: edetic acid 2Na | 0.8 | 0.8 | 0.5 |
| Antioxidant: propyl gallate | 0.2 | 0.2 | 0.01 |
| Antiseptic: methylparaben etc. | 0.20 | 0.06 | 0.06 |
| thickener: light anhydrous silicic acid | 1.0 | 1.0 | 0.25 |
| Ointment base: Plastibase (trade name) | 96.35 | 87.69 | 93.66 |
| Skin permeability: Qualitative evaluation test Quantitative evaluation test | + | + | + 0.2 (ng/layer) |
| Mouse transdermal absorbability test (delayed allergic reaction suppression rate) | 13.7% | 41.8% | 40.0% |
| [Note] •trietha: triethanolamine •diiso: diisopropanolamine •+ : Detected | | | |

From the results in Table 10, it was found that an ionic liquid for dissolution (levulinic acid triethanolamine salt) and an ionic liquid for dilution (isostearic acid diisopropanol amine salt) in the ointment preparation can be used in a content ratio ranging from 1:0 to 1:22, relative to the amount of ionic liquid for dissolution as 1. In particular, according to the mouse transdermal absorbability test, it was shown that when an ionic liquid for dilution offering low solubility of decoy is added to reduce the decoy solubility of the ionic liquid, the transdermal absorbability of the decoy further improves. Therefore, by using an ionic liquid for dissolution and an ionic liquid for dilution in combination, it became possible to select an ionic liquid offering appropriate solubility for the amount of decoy used.

Example 4: Effects of decoy concentration in ointment preparation on skin permeability

The influences of decoy concentration in ointment preparation on the skin permeability of NF-κB decoy were investigated. Reagents were weighed out to obtain the content ratios (w/w%) in Table 11 below, and a total of 50.00 g of decoy ointment preparation was prepared. The skin permeability (peeling test) and mouse transdermal absorbability (single-dose administration) of these ointment preparations were evaluated; the results are shown together in Table 11.

[Table 11]

| Test number | Production Example 1 | Preparation Example 5 |
|---|---|---|
| NF-κB decoy | 0.05 | 0.20 |
| Ionic liquid: | levulinic acid/trietha 0.4 | levulinic acid/trietha 1.6 |
| Solvent: propylene carbonate | 1.0 | 1.0 |
| Chelating agent: edetic acid 2Na | 0.8 | 0.8 |
| Antioxidant: propyl gallate | 0.2 | 0.2 |
| Antiseptic: methylparaben etc. | 0.20 | 0.06 |
| thickener: light anhydrous silicic acid | 1.0 | 1.0 |
| Ointment base: Plastibase (trade name) | 96.35 | 95.14 |
| Skin permeability: Qualitative evaluation test | + | + |
| Mouse transdermal absorbability test (delayed allergic reaction suppression rate) | 13.7% | 40.5% |
| [Note] •trietha: triethanolamine •+: Detected | | |

As shown in the results in Table 11, it was found that when the decoy concentration in the ionic liquid is constant, the decoy concentration in the ointment preparation largely influenced the transdermal absorbability.

Example 5: Effects of decoy concentration in ionic liquid on skin permeability

The skin permeability of NF-κB decoy is influenced by the decoy concentration in ointment preparation, as shown in Example 4, and, as shown in Example 3(2), the decoy concentration in ionic liquid (whether or not close to saturation concentration) has also a major influence. Hence, the influences of ionic liquid decoy concentration in the preparation on skin permeability were investigated. Reagents were weighed out to obtain the content ratios (w/w%) in Table 12 below, and a total of 50.00 g of decoy ointment preparation was prepared. The skin permeability (peeling test) of these ointment preparations was evaluated; the results are also shown in Table 12.

[Table 12]

| Test number | Production Example 1 | Production Example 6 | Production Example 7 | Production Example 8 |
|---|---|---|---|---|
| NF-κB decoy | 0.05 | 0.05 | 0.05 | 0.05 |
| Ionic liquid: levulinic acid/trietha (decoy concentration in ionic liquid) (degree of saturation) | 0.4 (12.5%) (44%) | 0.8 (5.9%) (21%) | 1.6 (3.0%) (11%) | 4.0 (1.2%) (4%) |
| Solvent: propylene carbonate | 1.0 | 1.0 | 1.0 | 1.0 |

(continued)

| Test number | Production Example 1 | Production Example 6 | Production Example 7 | Production Example 8 |
|---|---|---|---|---|
| Chelating agent: edetic acid 2Na | 0.8 | 0.8 | 0.8 | 0.8 |
| Antioxidant: propyl gallate | 0.2 | 0.2 | 0.2 | 0.2 |
| Antiseptic: methylparaben etc. | 0.20 | 0.20 | 0.20 | 0.20 |
| thickener: light anhydrous silicic acid | 1.0 | 1.0 | 1.0 | 1.0 |
| Ointment base: Plastibase (trade name) | 96.35 | 95.95 | 95.15 | 92.75 |
| Skin permeability: Qualitative evaluation test | + | N.D. | N.D. | N.D. |
| [Note] •trietha: triethanolamine • N.D.: Not Detected •+: Detected •Degree of saturation: (decoy concentration in ionic liquid in preparation) / (saturation solubility of ionic liquid) | | | | |

As shown in Table 12, it was shown that when the decoy concentration in the preparation (0.05%) is constant, the skin permeability of the decoy is largely influenced by the decoy concentration in the ionic liquid. Specifically, as shown by the results above, when the ionic liquid concentration in the preparation was 16 times or more higher than the decoy concentration in the preparation, the skin permeability was no longer detectable. From decoy solubility (Table 1), it is seen that to dissolve the decoy, the ionic liquid concentration is required to be about 3 times or more higher than the decoy concentration. For this reason, it was thought that the ionic liquid concentration required for dissolving the decoy and increasing the skin permeability thereof may be in the range between about 3 times and less than 16 times the decoy concentration in case of, for example, the levulinic acid triethanolamine salt.

Furthermore, because the saturation solubility is 28.3w/w% (Table 1) in case of the levulinic acid triethanolamine salt, the degrees of saturation in Production Example 1 and Production Example 6 are 44% and 21%, respectively. Hence, it was shown that when the degree of saturation in the ionic liquid is about 40% or more, the skin permeability becomes remarkable, and that when the degree of saturation is about 21% or less, the skin permeability disappears. This result is thought to be likewise applicable to other ionic liquids and mixed ionic liquids.

Example 6: Effects of decoy concentration in mixed ionic liquid on skin permeability

In external preparations, as the concentration of the drug dissolved in the base increases, the osmotic pressure due to the concentration works more, and the skin permeability improves. In Example 5, changes in the decoy concentration in a single ionic liquid were examined; it was found that the decoy concentration largely influences the skin permeability. Hence, an investigation was made to determine whether or not the same applied in case of mixed ionic liquid. First, using the NF-κB decoy shown by SEQ ID NO:1, decoy ointment preparations were prepared to obtain a total amount of 50 g according to the content ratios (w/w%) in Table 13 below in the same manner as Example 3. The mouse transdermal absorbability (single-dose administration) of the NF-κB decoy in these ointment preparations was evaluated; the results are shown in Tables 13 and 14 and Fig. 1.

[Table 13]

| Test number | Prep. Ex. 5 | Prep. Ex. 4 | Prep. Ex. 9 | Prep. Ex. 10 | Com. Ex. 2 |
|---|---|---|---|---|---|
| NF-κB decoy | 0.20 | 0.05 | 0.02 | 0.01 | 0 |
| Ionic liquid: levulinic acid/trietha | | | | | |

(continued)

| Test number | Prep. Ex. 5 | Prep. Ex. 4 | Prep. Ex. 9 | Prep. Ex. 10 | Com. Ex. 2 |
|---|---|---|---|---|---|
| isostearic acid/diiso | 1.6<br>0 | 0.8<br>3.67 | 0.8<br>3.67 | 0.8<br>3.67 | 0.8<br>3.67 |
| Solvent:<br>propylene carbonate | 1.0 | 1.0 | 1.0 | 1.0 | 1.0 |
| Chelating agent:<br>edetic acid 2Na | 0.8 | 0.5 | 0.5 | 0.5 | 0.5 |
| Antioxidant:<br>propyl gallate | 0.2 | 0.01 | 0.01 | 0.01 | 0.01 |
| Antiseptic:<br>methylparaben etc. | 0.06 | 0.06 | 0.06 | 0.06 | 0.06 |
| Thickener:<br>light anhydrous silicic acid | 1.00 | 0.25 | 0.25 | 0.25 | 0.25 |
| Ointment base:<br>Plastibase (trade name) | 95.14 | 93.66 | 93.69 | 93.70 | 93.71 |
| Mouse transdermal absorbability test (delayed allergic reaction suppression rate) | 40.5% | 40.0% | 37.4% | 15.6% | 0% |
| [Note]<br>•trietha: triethanolamine<br>•diiso: diisopropanolamine | | | | | |

[Table 14]

| Group | OVA | NF-$\kappa$B decoy (%) | Mean ($\times 10^{-3}$mm) | SE | Suppression rate versus 0% group (%) |
|---|---|---|---|---|---|
| Normal | - | 0 | -5.57 | 2.19 | - |
| 0% | + | 0 (Comparative Example 2) | 122.67 | 10.98 | 0 |
| 0.01% | + | 0.01 (Preparation Example 10) | 103.50 | 9.08 | 15.63 |
| 0.02% | + | 0.02 (Preparation Example 9) | 76.75 | 12.07 | 37.40 |
| 0.05% | + | 0.05 (Preparation Example 4) | 73.60 | 9.40 | 40.00 |
| 0.20% | + | 0.20 (Preparation Example 5) | 73.00 | 15.01 | 40.49 |

As shown in Tables 13 and 14 and Fig. 1, even when the decoy concentration in the ointment preparation was 0.01%, a tendency toward exhibiting good transdermal absorbability was noted. When the decoy concentration was 0.02%, 0.05% and 0.20%, a statistically significant therapeutic effect was exhibited, compared with the control (0%) ($p < 0.05$).

Compared with Preparation Examples 6 to 8 in Table 12, it is seen that the composition of ionic liquid (degree of decoy saturation in ionic liquid) has a major influence on the skin permeability of NF-$\kappa$B decoy. Specifically, as shown in Preparation Examples 6 to 8 in Table 12, with an ionic liquid offering high solubility of decoy (levulinic acid triethanolamine salt) only, skin permeability of the decoy was not found when the decoy concentration in the ionic liquid was about 6% or less,. However, in case of the mixed ionic liquids shown in Table 13 (Preparation Examples 4, 9, and 10), high skin permeability of the decoy was noted with dilution with an ionic liquid offering low solubility of decoy (isostearic acid diisopropanolamine salt), even when the decoy concentration in the ionic liquid was about 6% or less. Therefore, it was found that the skin permeability can be promoted by using a mixed ionic liquid, and choosing a ratio of an ionic liquid for dissolution (levulinic acid triethanolamine salt) and an ionic liquid for dilution (isostearic acid diisopropanolamine salt) depending on the decoy content in the preparation to have an appropriate setting of the degree of saturation.

Example 7: <u>Effects of decoy concentration in mixed ionic liquid-based ointment preparations</u>

With the concentration of mixed ionic liquid kept constant, an investigation was made to determine how changes in decoy concentration in the preparation influenced the skin permeability. Reagents were weighed out to obtain the content ratios (w/w%) in Table 15 below, and a total of 50.00 g of decoy ointment preparation was prepared according to Example 3. The skin permeability (peeling test) and mouse transdermal absorbability (single-dose administration) of these ointment preparations were evaluated; the results are also shown in Table 15.

[Table 15]

| Test number | Product.Ex. 12 | Product.Ex. 13 | Product.Ex. 14 | Product.Ex. 15 | Product.Ex. 16 |
|---|---|---|---|---|---|
| NF-κB decoy | 0.005 | 0.01 | 0.02 | 0.05 | 0.1 |
| Ionic liquid: | | | | | |
| levulinic acid/trietha | 0.8 | 0.8 | 0.8 | 0.8 | 0.8 |
| isostearic acid/diiso | 3.67 | 3.67 | 3.67 | 3.67 | 3.67 |
| Chelating agent: | | | | | |
| edetic acid 2Na | 0.5 | 0.5 | 0.5 | 0.5 | 0.5 |
| Antioxidant: | | | | | |
| propyl gallate | 0.01 | 0.01 | 0.01 | 0.01 | 0.01 |
| Sodium hydrogen sulfite | 0.001 | 0.001 | 0.001 | 0.001 | 0.001 |
| Antiseptic: | | | | | |
| methylparaben etc. | 0.06 | 0.06 | 0.06 | 0.06 | 0.06 |
| thickener: | | | | | |
| light anhydrous silicic acid | 0.25 | 0.25 | 0.25 | 0.25 | 0.25 |
| ointment base: Plastibase (trade name) | 94.70 | 94.69 | 94.66 | 94.61 | 94.11 |
| Skin permeability: Quantitative evaluation test (ng/layer) | 0.4 | 0.6 | 1.7 | 2.4 | 3.7 |
| Mouse transdermal absorbability test (delayed allergic reaction suppression rate) | - | 33.0 | 33.3 | 30.6 | 21.5 |
| [Note]<br>• trietha: triethanolamine<br>• diiso: diisopropanolamine<br>•-: Not examined | | | | | |

As shown in Table 15, according to the quantitative evaluation test of skin permeability (peeling test 3rd layer), the amount of decoy permeating the skin increased as the decoy concentration rose. Correspondingly, an effect was exhibited also in the mouse transdermal absorbability test (delayed allergic reaction suppression rate).

Example 8: <u>Effects of concentration of mixed ionic liquid in low-concentration decoy ointment preparations</u>

With the decoy concentration in the preparation fixed at low levels, an investigation was made to determine how changes in the concentration of mixed ionic liquid influenced the skin permeability. Reagents were weighed out to obtain the content ratios (w/w%) in Table 16 below, and a total of 50.00 g of decoy ointment preparation was prepared according to Example 3. The skin permeability (peeling test) and mouse transdermal absorbability (single-dose administration) of these ointment preparations were evaluated; the results are also shown in Table 16.

[Table 16]

| Test number | Production Example 17 | Production Example 18 | Production Example 12 |
|---|---|---|---|
| NF-κB decoy | 0.001 | 0.005 | 0.005 |
| Ionic liquid: | | | |
| levulinic acid/trietha | 0.016 | 0.08 | 0.8 |
| isostearic acid/diiso | 0.073 | 0.367 | 3.67 |
| Chelating agent: | | | |
| edetic acid 2Na | 0.5 | 0.5 | 0.5 |
| Antioxidant: | | | |
| propyl gallate | 0.007 | 0.01 | 0.01 |
| Sodium hydrogen sulfite | 0.00002 | 0.0001 | 0.001 |
| Antiseptic: | | | |
| methylparaben etc. | 0.06 | 0.06 | 0.06 |
| thickener: | | | |
| light anhydrous silicic acid | 0.25 | 0.25 | 0.25 |
| Ointment base: | | | |
| Plastibase (trade name) | 99.10 | 98.74 | 94.70 |
| Skin permeability: | | | |
| Quantitative evaluation test (ng/layer) | 0.3 | 3.0 | 0.4 |
| Mouse transdermal absorbability test (delayed allergic reaction suppression rate) | 9.0 | 13.0 | - |
| [Note] •trietha: triethanolamine •diiso: diisopropanolamine •-: Not examined | | | |

As shown in Table 16, it was shown that even when the NF-κB decoy concentration in the preparation is at an extremely low level of 0.001%, the decoy was transdermally absorbed and exhibited an effect.

Furthermore, according to the skin permeability (peeling test 3rd layer), as shown in Production Example 12 and Production Example 18, with the decoy concentration kept constant, the decoy skin permeability rose about 8 folds with a reduction of the amount of ionic liquid used to a one-tenth level to raise the decoy concentration in the ionic liquid. Hence, the decoy concentration in the ionic liquid makes a major contribution. For this reason, it is found that when the decoy concentration in the preparation was reduced with the decoy concentration in the ionic liquid kept constant, the skin permeability decreases to an about one-tenth level as the decoy concentration in the preparation is lowered to a one-fifth level, as shown in Production Example 17 and Production Example 18. Likewise, in the mouse transdermal absorbability test (delayed allergic reaction suppression rate), the effect decreased to two-thirds.

Example 9: Influences of changes in composition of mixed ionic liquid (changes in composition of ionic liquid for dissolution and ionic liquid for dilution) on skin permeability of decoy

To investigate how changes in the composition of mixed ionic liquid influenced the skin permeability, with the composition in Production Example 14 fixed, and only the ionic liquid for dilution (isostearic acid diisopropanolamine salt) and Plastibase (trade name) in the preparation changed, a total of 50.00 g of decoy ointment preparation (Preparation Examples 19 to 25) containing a mixed ionic liquid wherein the content ratio of the ionic liquid for dissolution and ionic liquid for dilution was 1:0 to 1:30 was prepared according to Example 3. The mouse transdermal absorbability (single-dose administration) of these ointment preparations was evaluated. The results are also shown in Table 17 below.

[Table 17]

| Prep. Ex. No. | NF-κB decoy (w/w%) | Ionic liquid blending ratio a) | isostearic acid/diiso (w/w%) | Mouse transdermal absorbability test (suppression rate) | Skin permeability: Quantitative evaluation test |
|---|---|---|---|---|---|
| 19 | 0.02 | 1:0 | 0 | 8.5 | - |
| 20 | 0.02 | 1:0.5 | 0.40 | 8.3 | - |
| 21 | 0.02 | 1:2 | 1.60 | 30.6* | - |
| 14 | 0.02 | 1:4.6 | 3.67 | 33.3* | 1.7 |
| 22 | 0.02 | 1:10 | 8.00 | 34.7* | - |
| 23 | 0.02 | 1:13 | 10.40 | 40.8* | - |
| 24 | 0.02 | 1:20 | 16.00 | 15.0 | - |
| 25 | 0.02 | 1:30 | 24.00 | 12.0 | - |

[Note]
•diiso: diisopropanolamine
•-: Not examined
• a): Levulinic acid triethanolamine:isostearic acid diisopropanolamine
• *: $p < 0.05$ (vs. 0% group)

As shown in Table 17 above, when a mixture of levulinic acid triethanolamine and isostearic acid diisopropanolamine was used as the ionic liquid, suppression versus the 0% group was observed in a broad range of content ratio of 1:0 to 1:30, with statistically significant suppression noted particularly at 1:2 to 1:13.

Example 10: Influences of combination of various ionic liquids on skin permeability of decoy

Mixed ionic liquids were prepared using various ionic liquids, and an investigation was made to determine how changes in the composition influenced the skin permeability. Reagents were weighed out to obtain the content ratios (w/w%) in Table 18 below, and a total of 50.00 g of decoy ointment preparation was prepared according to Example 3. The skin permeability (peeling test) and mouse transdermal absorbability (single-dose administration) of these ointment preparations were evaluated; the results are also shown in Table 18.

[Table 18]

| Preparation Example No. | Preparation Example 26 | Preparation Example 11 | Preparation Example 27 | Preparation Example 28 |
|---|---|---|---|---|
| NF-κB decoy | 0.05 | 0.05 | 0.05 | 0.01 |
| Ionic liquid: | levulinic acid/dietha 1.47 levulinic acid/triiso 4.41 | levulinic acid/trietha 0.93 levulinic acid/triiso 3.67 | levulinic acid/trietha 0.93 capric acid/diiso 0.23 | levulinic acid/trietha 0.32 isostearic acid/diiso 0.73 levulinic acid/triiso 0.35 |
| Chelating agent: edetic acid 2Na | 0 | 0.5 | 0. 5 | 0 |
| Antioxidant: propyl gallate Sodium hydrogen sulfite | 0.02 0.002 | 0.01 0.001 | 0.1 0.001 | 0.004 0.0004 |
| Antiseptic: methylparaben etc. | 0 | 0.06 | 0.06 | 0 |
| thickener: light anhydrous silicic acid | 0 | 0.25 | 0.25 | 0 |

(continued)

| Preparation Example No. | Preparation Example 26 | Preparation Example 11 | Preparation Example 27 | Preparation Example 28 |
|---|---|---|---|---|
| Ointment base: Plastibase (trade name) | 94.05 | 94.69 | 97.97 | 94.61 |
| Skin permeability: Quantitative evaluation test (ng/layer) | 5.1 | 1.0 | - | 11.6 |
| Mouse transdermal absorbability test (delayed allergic reaction suppression rate) | - | 34.1* | 24.9* | 35.3* |
| [Note]<br>•trietha: triethanolamine<br>•diiso: diisopropanolamine<br>• -: Not examined<br>• *: $p < 0.05$ (vs. 0% group) | | | | |

The mixed ionic liquid compositions in Table 18 were set at levels close to the saturation solubility, on the basis of the decoy concentrations used and the decoy solubilities in the various mixed ionic liquids shown in Example 2. As a result, skin permeability of the decoy was demonstrated in the skin permeability test (peeling test 3rd layer) and mouse transdermal absorbability test.

Hence, by designing an ionic liquid composition in consideration of the decoy solubility and decoy concentration in the ionic liquid, it became possible to transdermally absorb the decoy adequately.

Example 11: Repeated administration of preparation and effect to reduce decoy concentration in preparation

In a decoy skin permeability evaluation by single-dose administration of ointment preparations, results were obtained showing that even when the decoy concentration in the preparation was 0.001%, the decoy permeated the skin and had a suppressive effect on delayed allergic reactions, as shown in Example 8. For this reason, it is expected that by performing repeated administration, the decoy concentration can further be reduced. Hence, reagents were weighed out to obtain the content ratios (w/w%) in Table 19 below, and a total of 50.00 g of decoy ointment preparation was prepared according to Example 3. The mouse transdermal absorbabilities of the decoy in these ointment preparations were evaluated according to the above-described protocol for repeated administration. The results are also shown in Table 19.

[Table 19]

| Preparation Example No. | Preparation Example 29 | Preparation Example 30 | Preparation Example 31 | Preparation Example 32 |
|---|---|---|---|---|
| NF-$\kappa$B decoy | 0.0005 | 0.001 | 0.002 | 0.005 |
| Ionic liquid: levulinic acid/trietha | 0.80 | 0.80 | 0.80 | 0.80 |
| isostearic acid/diiso | 3.67 | 3.67 | 3.67 | 3.67 |
| (blending ratio) | 1:4.6 | 1:4.6 | 1:4.6 | 1:4.6 |
| Chelating agent: edetic acid 2Na | 0.5 | 0.5 | 0.5 | 0.5 |
| Antioxidant: propyl gallate | 0.01 | 0.01 | 0.1 | 0.01 |
| Sodium hydrogen sulfite | 0.001 | 0.001 | 0.001 | 0.001 |
| Antiseptic: methylparaben etc. | 0.06 | 0.06 | 0.06 | 0.06 |
| thickener: | | | | |

(continued)

| Preparation Example No. | Preparation Example 29 | Preparation Example 30 | Preparation Example 31 | Preparation Example 32 |
|---|---|---|---|---|
| light anhydrous silicic acid | 0.25 | 0.25 | 0.25 | 0.25 |
| Ointment base:<br>Plastibase (trade name) | 94.71 | 94.71 | 97.71 | 94.70 |
| Mouse transdermal absorbability test (delayed allergic reaction suppression rate) | 26.2 | 20.8 | 28.3* | 21.7 |
| •trietha: triethanolamine<br>•diiso: diisopropanolamine<br>• *: $p < 0.05$ (vs. 0% group) | | | | |

As shown in Table 19, when an ointment preparation was repeatedly administered, statistically significant suppression was noted at an NF-κB decoy concentration of 0.002%, with suppression also observed at the other concentrations. Since efficacy in delayed allergic reaction suppression was noted even at an NF-κB decoy concentration lower than that for single-dose administration (0.0005%), it was found that by performing repeated administration, the decoy concentration used can further be reduced.

Industrial Applicability

Because the external preparation composition of the present invention offers excellent solubility and transdermal absorbability of transcription factor decoys, it can serve as a convenient and effective DDS preparation for transcription factor decoys. Because the external preparation composition of the present invention also offers extremely excellent transdermal absorbability even at a decoy concentration that is about 1/10000 of a level currently in a clinical study (2%) with the use of a mixed ionic liquid, the external preparation composition of the present invention can significantly reduce the cost of decoy type nucleic acid drugs to contribute to medical economics.

**Claims**

1.  A nonaqueous external preparation composition containing a transcription factor decoy dissolved in a fatty acid-based ionic liquid obtained from a fatty acid having 2 to 20 carbon atoms and an organic amine compound having 4 to 12 carbon atoms, wherein the transcription factor decoy is a double-stranded oligonucleotide consisting of 14 to 35 base pairs and selected from the group consisting of decoys for NF-κB, E2F, GATA-3, STAT-1, STAT-6, Ets and AP-1.

2.  The non-aqueous external preparation composition according to claim 1, wherein the transcription factor decoy consists of 17 to 25 base pairs.

3.  The non-aqueous external preparation composition according to claim 1 or 2, wherein the transcription factor decoy is NF-κB decoy.

4.  The nonaqueous external preparation composition according to any one of claims 1 to 3, wherein the fatty acid having 2 to 20 carbon atoms is one or more selected from the group consisting of glycolic acid, methoxyacetic acid, levulinic acid, capric acid and isostearic acid.

5.  The non-aqueous external preparation composition according to any one of claims 1 to 4, wherein the organic amine compound having 4 to 12 carbon atoms is one or more selected from the group consisting of diethanolamine, triethanolamine, diisopropanolamine and triisopropanolamine,

6. The non-aqueous external preparation composition according to any one of claims 1 to 5, wherein the fatty acid-based ionic liquid is a mixed ionic liquid of a plurality of fatty acid-based ionic liquids.

7. The non-aqueous external preparation composition according to claim 6, wherein the mixed ionic liquid is a mixture of an ionic liquid (I) that is the diethanolamine salt or triethanolamine salt of a fatty acid having 2 to 5 carbon atoms and an ionic liquid (II) that is the diisopropanolamine salt or triisopropanolamine salt of a fatty acid having 2 to 20 carbon atoms.

8. The non-aqueous external preparation composition according to claim 7, wherein the fatty acid having 2 to 5 carbon atoms of the ionic liquid (I) is levulinic acid.

9. The non-aqueous external preparation composition according to claim7 or 8, wherein the fatty acid having 2 to 20 carbon atoms of the ionic liquid (II) is isostearic acid

10. The non-aqueous external preparation composition according to claim 9, wherein the ratio by weight of the ionic liquid (I) and the ionic liquid (II) ranges from 1:1 to 1:50.

11. The non-aqueous external preparation composition according to any one of claim 1 to 10, wherein the concentration of the transcription factor decoy is 0.0001 to 1 w/w %.

12. The non-aqueous external preparation composition according to any one of claims 1 to 11, wherein
an ionic liquid that is the diethanolamine salt or triethanolamine salt of a fatty acid having 2 to 5 carbon atoms is contained at a concentration 3 to 1000 times the concentration of the transcription factor decoy, and
a mixed ionic liquid of a plurality of fatty acid-based ionic liquids is contained, the concentration of the mixed ionic liquid being 0.008 to 30 w/w %.

13. The non-aqueous external preparation composition according to any one of claims 1 to 12, wherein a chelating agent is added.

14. The nonaqueous external preparation composition according to any one of claim 1 to 13, wherein the composition is in the form of an ointment with the addition of an ointment base.

15. The non-aqueous external preparation composition according to any one of claim 1 to 14, wherein the NF-κB decoy comprises the sequence GGRHTYYHC (wherein R stands for A or G, Y for C or T, and H for A, C or T).

16. The non-aqueous external preparation composition according to any one of claim 1 to 14, wherein the NF-κB decoy comprises the sequence GGATTTCCC or GGACTTTCC.

**Patentansprüche**

1. Nicht.wässrige Zusammensetzung zur topischen Anwendung enthaltend einen Transkriptionsfaktor-Decoy gelöst in einer ionischer Flüssigkeit auf der Grundlage einer Fettsäure, erhalten von einer 2 bis 20 Kohlenstoffatome enthaltenden Fettsäure und einer 4 bis 12 Kohlenstoffatome enthaltenden organischem Aminoverbindung, wobei der Transkriptionsfaktor-Decoy ein Doppelstrang-Oligonukleotid ist, welcher aus 14 bis 35 Basenpaaren besteht und ausgewählt wird aus der Gruppe bestehend aus den Decoys für NF-κB, E2F, GATA-3, STAT-1, STAT-6, Ets und AP-1.

2. Die nicht-wässrige Zusammensetzung zur topischen Anwendung gemäß Anspruch 1, wobei der Transkriptionsfaktor-Decoy aus 17 bis 25 Basenpaaren besteht.

3. Die nicht-wässrige Zusammensetzung zur topischen Anwendung gemäß Anspruch 1 oder 2, wobei der Transkriptionsfaktor-Decoy der Decoy für NF-κB ist.

4. Die nicht-wässrige Zusammensetzung zur topischen Anwendung gemäß einem der Ansprüche 1 bis 3, wobei die 2 bis 20 Kohlenstoffatome enthaltende Fettsäure ausgewählt wird aus der Gruppe bestehend aus Glycolsäure, Methoxyessigsäure, Lävulinsäure, n-Caprinsäure und Isostearinsäure.

**5.** Die nicht-wässrige Zusammensetzung zur topischen Anwendung gemäß einem der Ansprüche 1 bis 4, wobei die 4 bis 12 Kohlenstoffatome enthaltende organische Aminoverbindung eine oder mehrere ausgewählt aus der Gruppe bestehend aus Diethanolamin, Triethanolamin, Diisopropanolamin und Triisopropanolamin ist.

**6.** Die nicht-wässrige Zusammensetzung zur topischen Anwendung gemäß einem der Ansprüche 1 bis 5, wobei die ionische Flüssigkeit auf der Grundlage einer Fettsäure eine gemischte ionische Flüssigkeit aus einer Vielzahl von ionischen Flüssigkeiten auf der Grundlage einer Fettsäure ist.

**7.** Die nicht-wässrige Zusammensetzung zur topischen Anwendung gemäß Anspruch 6, wobei die gemischte ionische Flüssigkeit eine Mischung ist aus einer ionischen Flüssigkeit (I), welche das Salz einer 2 bis 5 Kohlenstoffatome enthaltenden Fettsäure mit Diethanolamin oder Triethanolamin ist, und einer ionischen Flüssigkeit (II), welche das Salz einer 2 bis 20 Kohlenstoffatome enthaltenden Fettsäure mit Dlisopropanolamin oder Triisopropanolamin ist.

**8.** Die nicht-wässrige Zusammensetzung zur topischen Anwendung gemäß Anspruch 7, wobei die 2 bis 5 Kohlenstoffatome enthaltende Fettsäure der ionischen Flüssigkeit (I) Lävulinsäure ist.

**9.** Die nicht-wässrige Zusammensetzung zur topischen Anwendung gemäß Anspruch 7 oder 8, wobei die 2 bis 20 Kohlenstoffatome enthaltende Fettsäure der ionischen Flüssigkeit (II) Isostearinsäure ist.

**10.** Die nicht-wässrige Zusammensetzung zur topischen Anwendung gemäß Anspruch 9, wobei das Gewichtsverhältnis der ionischen Flüssigkeit (I) und der ionischen Flüssigkeit (II) von 1:1 bis 1:50 reicht.

**11.** Die nicht-wässrige Zusammensetzung zur topischen Anwendung gemäß irgendeinem der Ansprüche 1 bis 10, wobei die Konzentration des Transkriptionsfaktor-Decoys 0,0001 bis 1 w/w % betragt.

**12.** Die nicht-wässrige Zusammensetzung zur topischen Anwendung gemäß irgendeinem der Ansprüche 1 bis 11, wobei eine ionische Flüssigkeit, welche das Salz einer 2 bis 5 Kohlenstoffatome enthaltenden Fettsäure mit Diethanolamin oder Triethanolamin ist, in einer Konzentration von 3 bis 1000 Mal die Konzentration des Transkriptionsfaktor-Decoys enthalten ist, und

eine gemischte ionische Flüssigkeit aus einer Vielzahl von Fettsäure- basierten ionischen Flüssigkeiten enthalten ist und dabei due Konzentration der gemischten ionischen Flüssigkeit 0,008 bis 30 w/w% beträgt.

**13.** Die nicht-wässrige Zusammensetzung zur topischen Anwendung gemäß irgendeinem der Ansprüche 1 bis 12, wobei ein Chelatbildner hinzugefügt wird.

**14.** Die nicht-wässrige Zusammensetzung zur topischen Anwendung gemäß irgendeinem der Ansprüche 1 bis 13, wobei die Zusammensetzung In der Form einer Salbe ist, mit Zugabe einer Salbengrundlage.

**15.** Die nicht-wässrige Zusammensetzung zur topischen Anwendung gemäß irgendeinem der Ansprüche 1 bis 14, wobei der Decoy für NF-κB die Sequenz GGRHTYYHC umfasst (wobei R für A oder G steht, Y für C oder T, und H für A, C oder T).

**16.** Die nicht-wässrige Zusammensetzung zur topischen Anwendung gemüß Irgendeinem der Ansprüche 1 bis 14, wobei der Decoy für NF-κB die Sequenz GGATTTCCC oder GGACTTTCC umfasst.

**Revendications**

**1.** Composition non aqueuse pour application externe contenant un leurre pour des facteurs de transcription dissous dans un liquide ionique à base d'acide gras obtenu à partir d'un acide gras comportant 2 à 20 atomes de carbone et un composé d'amine organique comportant 4 à 12 atomes de carbone, le leurre pour des facteurs de transcription étant un oligonucléotide double brin de 14 à 35 paires de bases et choisi dans le groupe constitué de leurres pour les NF-κB, E2F, GATA-3, STAT-1, STAT-6, Ets et AP-1.

**2.** Composition non aqueuse pour application externe selon la revendication 1, dans laquelle le leurre pour des facteurs de transcription est constitué de 17 à 25 paires de bases.

**3.** Composition non aqueuse pour application externe selon les revendications 1 ou 2, dans laquelle le leurre pour des facteurs de transcription est un leurre pour le NF-κB.

4. Composition non aqueuse pour application externe selon l'une quelconque des revendications 1 à 3, dans laquelle l'acide gras comportant 2 à 20 atomes de carbone est un ou plusieurs choisis dans le groupe constitué de l'acide glycolique, l'acide méthoxyacétique, l'acide lévulinique, l'acide caprique et l'acide isostéarique.

5. Composition non aqueuse pour application externe selon l'une quelconque des revendications 1 à 4, dans laquelle le composé d'amine organique comportant 4 à 12 atomes de carbone est un ou plusieurs choisis dans le groupe constitué de la diéthanolamine, la triéthanolamine, la diisopropanolamine et la triisopropanolamine.

6. Composition non aqueuse pour application externe selon l'une quelconque des revendications 1 à 5, laquelle le liquide ionique à base d'acide gras est un liquide ionique mixte de plusieurs liquides ioniques à base d'acides gras.

7. Composition non aqueuse pour application externe selon la revendication 6, dans laquelle le liquide ionique mixte est un mélange d'un liquide ionique (I) qui est le sel de diéthanolamine ou le sel de triéthanolamine d'un acide gras comportant 2 à 5 atomes de carbone et d'un liquide ionique (II) qui est le sel de diisopropanolamine ou le sel de triisopropanolamine d'un acide gras comportant 2 à 20 atomes de carbone.

8. Composition non aqueuse pour application externe selon la revendication 7, dans laquelle l'acide gras comportant 2 à 5 atomes de carbone du liquide ionique (I) est l'aaide lévulinique.

9. Composition non aqueuse pour application externe selon les revendications 7 ou 8, dans laquelle l'acide gras comportant 2 à 20 atomes de carbone du liquide ionique (II) l'acide isostéarique.

10. Composition non aqueuse pour application externe selon la revendication 9, dans laquelle le rapport en poids du liquide ionique (I) et du liquide ionique (II) va de 1/1 à 1/50.

11. Composition non aqueuse pour application externe selon l'une quelconque des revendications 1 à 10, dans laquelle la concentration du leurre pour des facteurs de transcription est de 0,0001 à 1 % p/p.

12. Composition non aqueuse pour application externe selon l'une quelconque des revendications 1 à 11, dans laquelle un liquide ionique qui est le sel de diéthanolamine ou le sel de triéthanolamine d'un acide gras comportant 2 à 5 atomes de carbone est contenu à une concentration de 3 à 1000 fois la concentration du leurre pour des facteurs de transcription, et
un liquide ionique mixte de plusieurs liquides ioniques à base d'acides gras est contenu, la concentration du liquide ionique mixte étant de 0,008 à 30 % p/p.

13. Composition non aqueuse pour application externe selon l'une quelconque des revendications 1 à 12, dans laquelle un agent de chélation est ajouté.

14. Composition non aqueuse pour application externe selon l'une quelconque des revendications 1 à 13, la composition étant sous la forme d'une pommade avec l'addition d'une base de pommade.

15. Composition non aqueuse pour application externe selon l'une quelconque des revendications 1 à 14, dans laquelle le leurre pour le NF-κB comprend la séquence GGRHTYYHC (où R représente A ou G, Y représente C ou T, et H représente A, C ou T).

16. Composition non aqueuse pour application externe selon l'une quelconque des revendications 1 à 14, où le leurre pour le NF-κB comprend la séquence GGATTTCCC ou GGACTTTCC.

# FIG. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1362600 A1
- JP 8035430 A
- JP 3392143 B
- WO 9511687 A
- JP 2006089475 A
- JP 2007137891 A
- JP 2008056611 A
- JP 2007018004 A
- JP 2008088801 A

**Non-patent literature cited in the description**

- *Japanese Journal of Clinical Medicine,* 2005, vol. 12 (63), 659-663


SEQUENCE LISTING

<110> AnGesMG, Inc. MEDRx Co., Ltd.

<120> Composition for External Use Containing Transcription Factor Decoy as Active Ingredient

<130> 091384

<150> JP 2008-088801
<151> 2008-03-28

<160> 13

<170> PatentIn version 3.5

<210> 1
<211> 20
<212> DNA
<213> Artificial Sequence

<220>
<223> NF-kappa B decoy

<400> 1
ccttgaaggg atttccctcc        20

<210> 2
<211> 22
<212> DNA
<213> Artificial Sequence

<220>
<223> NF-kappa B decoy

<400> 2
agttgagggg actttcccag gc          22

<210> 3
<211> 19
<212> DNA
<213> Artificial Sequence

<220>
<223> NF-kappa B decoy

<400> 3
agttgaggac tttccaggc          19

<210> 4
<211> 14
<212> DNA
<213> Artificial Sequence

<220>
<223> E2F decoy

<400> 4
ctagatttcc cgcg          14

<210> 5
<211> 18
<212> DNA
<213> Artificial Sequence

<220>
<223> E2F decoy

<400> 5
ctagatttcc cgcggatc          18

<210> 6
<211> 16
<212> DNA
<213> Artificial Sequence

<220>
<223> GATA-3 decoy

<400> 6
agcttgagat agagct          16

<210> 7
<211> 28
<212> DNA

<213> Artificial Sequence

<220>
<223> STAT-1 decoy

<400> 7
gatctaggga tttccgggaa atgaagct          28

<210> 8
<211> 18
<212> DNA
<213> Artificial Sequence

<220>
<223> STAT-1 decoy

<400> 8
tgtgaattac cggaagtg          18

<210> 9
<211> 28
<212> DNA
<213> Artificial Sequence

<220>
<223> STAT-6 decoy

<400> 9
gatcaagacc ttttcccaag aaatctat          28

<210> 10
<211> 20
<212> DNA
<213> Artificial Sequence

<220>
<223> Ets decoy

<400> 10
aattcaccgg aagtattcga          20

<210> 11
<211> 19
<212> DNA
<213> Artificial Sequence

<220>
<223> AP-1 decoy

<400> 11
agcttgtgag tcagaagct          19

<210> 12
<211> 10
<212> DNA
<213> Artificial Sequence

<220>

<223> STAT-6-binding consensus sequence

<220>
<221> misc_feature
<222> (4) .. (4)
<223> n stands for a, g, c or t

<220>
<221> misc_feature
<222> (5) .. (5)
<223> n stands for a, g, c or t

<220>
<221> misc_feature
<222> (6) .. (6)
<223> n stands for a, g, c or t

<220>
<221> misc_feature
<222> (7) .. (7)
<223> n stands for a, g, c or t

<400> 12
ttcnnnngaa          10

<210> 13
<211> 10
<212> DNA
<213> Artificial Sequence

<220>
<223> STAT-6-binding consensus sequence

<400> 13
ttcccaagaa          10